# EUROPEAN PATENT SPECIFICATION

(11) **EP 3 538 158 B1**
(45) Date of publication and mention of the grant of the patent: **01.11.2023**
(21) Application number: 17830003.4
(22) Date of filing: 10.11.2017
(51) Int. Cl.: A61K 47/54, A61K 45/06, A61P 1/16

(54) **TREATMENT FOR FIBROSIS**
BEHANDLUNG VON FIBROSE
TRAITEMENT DE LA FIBROSE

(30) Priority: 10.11.2016 US 201662420009 P; 10.11.2016 US 201662420017 P; 10.11.2016 US 201662420012 P; 22.03.2017 US 201762475129 P
(43) Date of publication of application: 18.09.2019
(73) Proprietor: Galmed Research and Development Ltd., 6578317 Tel Aviv (IL)
(72) Inventor: HAYARDENY-NISSIMOIV, Liat, 69391 Tel Aviv (IL); GORFINE, Tali, 6494210 Tel Aviv (IL); BAHARAFF, Allen, 66215441 Tel Aviv (IL); MATO DE LA PAZ, José, M., 48992 Getxo (ES)
(74) Representative: Pearl Cohen Zedek Latzer Baratz UK LLP
(86) International application number: PCT/IB2017/001521
(87) International publication number: WO 2018/087599

(56) References cited:
- WO-A1-2016/112305
- WO-A1-2017/017677
- WO-A1-2017/210526
- US-A1- 2011 014 126
- RIFAAT SAFADI ET AL: "The Fatty Acid-Bile Acid Conjugate Aramchol Reduces Liver Fat Content in Patients With Nonalcoholic Fatty Liver Disease", CLINICAL GASTROENTEROLOGY AND HEPATOLOGY, vol. 12, no. 12, 9 May 2014 (2014-05-09), pages 2085-2091.e1, XP55350035, US ISSN: 1542-3565, DOI: 10.1016/j.cgh.2014.04.038
- M. IRUARRIZAGA-LEJARRETA ET AL: "Role of Aramchol in Steatohepatitis and Fibrosis in Mice", HEPATOLOGY COMMUNICATIONS, vol. 1, no. 9, 3 November 2017 (2017-11-03), pages 911-927, XP002778683,

## Description

### BACKGROUND OF THE INVENTION

### Fibrosis

The formation of fibrous connective tissue is part of the normal healing process following tissue damage due to injury or inflammation. During this process, activated immune cells including macrophages stimulate the proliferation and activation of fibroblasts, which in turn deposit connective tissue. However, abnormal or excessive production of connective tissue may lead to accumulation of fibrous material such that it interferes with the normal function of the tissue. Fibrotic growth can proliferate and invade healthy surrounding tissue, even after the original injury heals. Such abnormal formation of excessive connective tissue, occurring in a reparative or reactive process, is referred to as fibrosis.

Physiologically, fibrosis acts to deposit connective tissue, which can obliterate the architecture and function of the underlying organ or tissue. Defined by the pathological accumulation of extracellular matrix (ECM) proteins, fibrosis results in scarring and thickening of the affected tissue, which interferes with normal organ function. In various conditions, the formation of fibrotic tissue is characterized by the deposition of abnormally large amounts of collagen. The synthesis of collagen is also involved in a number of other pathological conditions. For example, clinical conditions and disorders associated with primary or secondary fibrosis, such as systemic sclerosis, graft-versus host disease (GVHD), pulmonary fibrosis and autoimmune disorders, are distinguished by excessive production of connective tissue, which results in the destruction of normal tissue architecture and function. These diseases can best be interpreted in terms of perturbations in cellular functions, a major manifestation of which is excessive collagen synthesis and deposition. The role of collagen in fibrosis has prompted attempts to develop drugs that inhibit its accumulation.

### Hepatic Fibrosis

Fibrosis of the liver, also referred to herein as hepatic fibrosis, may be caused by various types of chronic liver injury, especially if an inflammatory component is involved. Self-limited, acute liver injury (e.g., acute viral hepatitis A), even when fulminant, does not necessarily distort the scaffolding architecture and hence does not typically cause fibrosis, despite loss of hepatocytes. However, factors such as chronic alcoholism, malnutrition, hemochromatosis, and exposure to poisons, toxins or drugs, may lead to chronic liver injury and hepatic fibrosis due to exposure to hepatotoxic chemical substances. Hepatic scarring, caused by surgery or other forms of injury associated with mechanical biliary obstruction, may also result in liver fibrosis.

Fibrosis itself is not necessarily symptomatic, however it can lead to the development of portal hypertension, in which scarring distorts blood flow through the liver, or cirrhosis, in which scarring results in disruption of normal hepatic architecture and liver dysfunction. The extent of each of these pathologies determines the clinical manifestation of hepato-fibrotic disorders. For example, congenital hepatic fibrosis affects portal vein branches, largely sparing the parenchyma. The result is portal hypertension with sparing of hepatocellular function.

### Treatment

Attempts to develop anti-fibrotic agents for the treatment of various disorders have been reported. However, treatment of established fibrosis, formed after months or years of chronic or repeated injury, still remains a challenge. In its initial stages, hepatic fibrosis may regress if the cause is reversible (e.g. with viral clearance). Thus, the majority of available treatment options are designed to remove the basis of the liver injury, such as by eliminating hepatitis B virus or hepatitis C virus in chronic viral hepatitis, abstaining from alcohol in alcoholic liver disease, removing heavy metals such as iron in hemochromatosis or copper in Wilson disease, and decompressing bile ducts in biliary obstruction.

Treatments aimed at reversing the fibrosis are usually too toxic for long-term use (e.g. corticosteroids, penicillamine) or have no proven efficacy (e.g. colchicine). Silymarin, present in milk thistle, is a popular alternative medicine used to treat hepatic fibrosis, appears to be safe but to lack efficacy.

### Potential Therapeutic agents

Attempts to develop specific anti-fibrotic agents for the treatment of liver diseases have been reported. For example, U.S. Pat. No. 8,729,046 relates to methods for treating fibrosis of a tissue, including fibrosis of the liver, using combinations of nucleic acids or nucleic acid analogs. Specifically, the nucleic acids or analogs thereof are targeted to microRNAs of the miR23b cluster. U.S. 6,562,829 discloses compositions for treating hepatic fibrosis and methods of using and manufacturing the composition, the composition comprising a quinazolinone derivative, preferably Halofuginone. U.S. 8,858,954 is directed to pharmaceutical composition for preventing and treating liver fibrosis or nonalcoholic fatty liver disease, comprising 50 to 90% by weight of Cordyceps sinensis mycelium powder, and 10 to 50% by weight of condensed astragalus powder.

U.S. Pub. No. 2015/359805 relates to Farnesoid X receptor (FXR) modulators which can be used for the treatment of cholestatic disorders, in particular to bile acid derivatives wherein the C6 contains an ethyl and the C24 carboxy group is transformed into a sulphate group. Among the disorders suggested to be treated are alcoholic liver disease, living donor transplant liver regeneration, congenital hepatic fibrosis, choledocholithiasis, and granulomatous liver disease. U.S. 2014/187633 is directed to methods of treating and/or preventing non-alcoholic seatohepatitis (NASH) and/or primary biliary cirrhosis comprising administering to a subject in need thereof a pharmaceutical composition comprising eicosapentaenoic acid or a derivative thereof. The FXR agonist, obeticholic acid, which is a modified bile acid, is in phase III clinical trials for primary biliary cirrhosis. Use of this drug has been reported to be commonly associated with side effects such as pruritus.

Ursodeoxycholic acid (UDCA, Ursodiol) is the most frequently used treatment for primary biliary cirrhosis. It is one of the secondary bile acids, which are metabolic byproducts of intestinal bacteria. The drug is considered to assist in reducing the cholestasis and improves blood test results (liver function tests). However it has a minimal effect on symptoms and whether it improves prognosis is controversial. To relieve itching caused by bile acids in circulation, which would normally be removed by the liver, cholestyramine (a bile acid sequestrant) may be prescribed to primary biliary cirrhosis patients. The agent may assist in absorbing bile acids in the gut to be eliminated, rather than re-enter the blood stream. Alternative agents include stanozolol, naltrexone and rifampicin.

Obeticholic acid (OCA, Ocaliva) is a semi-synthetic bile acid analogue undergoing development in phase 2 and 3 studies for specific liver and gastrointestinal conditions. The FDA granted accelerated approval to Ocaliva on 27 May 2016 for the treatment of primary biliary cholangitis (PBC) in combination with ursodeoxycholic acid (UDCA) in adults with an inadequate response to UDCA, or as a single therapy in adults unable to tolerate UDCA. In addition, a phase 2 trial in NASH patients showed that administration of OCA reduced markers of liver inflammation and fibrosis and increased insulin sensitivity.

WO 2014/197738 and WO 2016/094570 relate to small molecule compounds, disclosed to be inhibitors of myofibroblast trans-differentiation and activation. Drugs and combinations suggested for the treatment of *inter alia* fatty liver were disclosed, for example, in WO 2016/112305 and EP2632925 (acetyl-CoA carboxylase inhibitors) as well as WO 2016/154258 (dual PPAR delta / gamma agonists). Some of the disclosed compounds were suggested to be used in combination with various other drugs.

Many patients do not respond to available treatments for fibrotic disorders, and long-term treatment is limited by toxicity and side effects. Therefore, a need remains for developing therapeutic modalities aimed at reducing fibrosis, especially hepatic fibrosis. The development of safe and effective treatments for established cirrhosis and portal hypertension and for attenuating fibrosis would be highly beneficial.

### SUMMARY OF THE INVENTION

The present invention provides an oral dose of between 350 mg and 1200 mg per day of 3β-arachidylamido-7α, 12α-dihydroxy-5β-cholan-24-oic acid (Aramchol), or a pharmaceutically acceptable salt thereof, as the sole active ingredient for use in treating hepatic fibrosis in a human subject by down regulation of collagen production in the liver of the subject.

In an embodiment, the human subject is afflicted with Non-Alcoholic Fatty Liver Disease (NAFLD).

In an embodiment, the human subject is afflicted with Non-Alcoholic Steatohepatitis (NASH).

In another embodiment, the human subject is not afflicted with Non-Alcoholic Steatohepatitis (NASH).

In an embodiment, the human subject has a NAFLD Activity (NAS) Score of at least 4, at least 5, at least 6, or at least 7, or the human subject has a ballooning score of at least 1, an inflammation score of at least 1, and a steatosis score of at least 1.

In an embodiment, the human subject is afflicted with Diabetes Mellitus type II or p re-d i abetes.

In an embodiment, the hepatic fibrosis is stage 1, 2, 3, or 4 fibrosis.

In an embodiment, the oral dose is 400 mg, 600 mg or 800 mg of Aramchol per day.

In an embodiment, the Aramchol is administered over the course of at least 52 weeks, at least 72 weeks, at least 96 weeks, at least 2 years, at least 3 years, or at least 4 years.

In an embodiment, the treating comprises inhibiting progression of Non-Alcoholic Fatty Liver Disease (NAFLD) and/or Non-Alcoholic Steatohepatitis (NASH).

In an embodiment, the human subject is not afflicted with Non-Alcoholic Steatohepatitis (NASH) at commencement of administration and the treating comprises preventing progression from Non-Alcoholic Fatty Liver Disease (NAFLD) to NASH. In another embodiment, the human subject is afflicted with Non-Alcoholic Steatohepatitis (NASH) and the treating comprises NASH resolution in the subject, for example wherein NASH resolution comprises the human subject having a ballooning score of 0 and an inflammation score of 0 or 1.

In an embodiment, the treating comprises a reduction in the ratio of liver triglycerides to water in the subject relative to the ratio at the commencement of administration of Aramchol as measured by MRS, for example a 10% to 40% reduction in ratio of liver triglycerides to water.

In another embodiment, the treating comprises:
a. a reduction in the level of Hemoglobin A1C or HOMA-IR;
b. a reduction in the level of Fibrinogen, CK-18, C-reactive protein (CRP), TNFα, IL 6 and fibrosis Tests (NFS);
c. a reduction in the ratio of leptin to adiponectin; or
d. an increase in the level of adiponectin;
   in the subject relative to the level or ratio at the commencement of administration of Aramchol, or
e. a reduction in the human subject's body weight relative to the human subject's body weight at the commencement of administration of Aramchol;
f. a reduction in the human subject's waist circumference relative to the human subject's waist circumference at the commencement of administration of Aramchol; or
g. a reduction in the human subject's Fatty Liver Index relative to the human subject's Fatty Liver Index at the commencement of administration of Aramchol.

In an embodiment, the human subject has a diet that is high fat and high calorie; and/or is resistant to lifestyle intervention or is resistant to diet intervention, wherein a high fat meal is a meal wherein approximately 500 to 600 calories are fat calories and a high calorie meal is a meal of approximately 800 to 1000 calories.

The present invention also provides an oral dose of between 350 mg and 1200 mg per day of 3β-arachidylamido-7α, 12α-dihydroxy-5β-cholan-24-oic acid (Aramchol), or a pharmaceutically acceptable salt thereof, for use in treating hepatic fibrosis in a human subject by down regulation of collagen production in the liver of the subject, and the subject is further administered a therapeutically effect amount of at least one compound selected from a CCR2 chemokine antagonist and a CCR5 chemokine antagonist.

In an embodiment, the subject is afflicted with hepatic fibrosis and NASH and has a NAS Score of at least 5, at least 6, or at least 7, a ballooning score of at least 1, an inflammation score of at least 1, and a steatosis score of at least 1, and the subject is administered a dose of 400 mg or 600 mg or 800 mg per day of the Aramchol or salt thereof.

### BRIEF DESCRIPTION OF THE DRAWINGS

Figure 1. Demonstrates the effect of Aramchol on liver cirrhosis by macroscopic evaluation. Figure 1A - saline control; Figure 1B - treatment with TAA (20mg/100 gr body weight) twice per week during 10 weeks; Figure 1C - treatment with TAA and Aramchol 1 mg/kg; Figure 1D - treatment with TAA and Aramchol 5 mg/kg.
Figure 2. Demonstrates the effect of Aramchol on liver fibrosis by microscopic evaluation (following Masson Goldner staining). Figure 2A - averaged fibrotic score (TAA - black, Aramchol - white, OCA - gray); Figure 2B - representative samples (TAA only - left; TAA and Aramchol 1 mg/kg - middle; TAA and Aramchol 5 mg/kg - right).
Figure 3. Depicts the effect of Aramchol on COL1A1 expression in LX-2 human hepatic stellate cells. "Ctrl S1, S2 and S3" represent control (saline-treated cells) in three separate experiments; "A S1, S2 and S3" represent the result of Aramchol treated cells in these experiments.
Figure 4. Depicts the effect of Aramchol on PPAR-y expression in LX-2 human hepatic stellate cells. "Ctrl S1, S2 and S3" represent control (saline-treated cells) in three separate experiments; "A S1, S2 and S3" represent the result of Aramchol treated cells in these experiments.
Figure 5. Depicts the effect of Aramchol on collagen production from LX-2 human hepatic stellate cells compared to a DMSO control.
Figure 6. Depicts the effect of Aramchol on liver steatosis in 0.1 MCD diet. Figure 6A - histology staining using Sudan III; Figure 6B - quantification of Sudan III stained cells.
Figure 7. Depicts the effect of Aramchol on macrophage activation and infiltration in 0.1 MCD diet. Figure 7A - histology staining - F4/80 and CD64; Figure 7B - quantification of F4/80 and CD64 positive cells.
Figure 8. Depicts the effect of Aramchol on fibrosis in 0.1MCD Diet (histology- sirius red). Figure 8A - histology staining using sirius red; Figure8B - quantification of sirius red stained cells.
Figure 9. Depicts the effect of Aramchol on collagen production using liver extract from 0.1 MCD mice.
Figure 10. Depicts the effects of Aramchol on liver biochemistry in 0.1 MCD mice. Figure 10A - quantification of metabolites in liver of control (grey) and Aramchol-treated (black) 0.1 MCD mice; Figure 10B - schematic of relevant liver biochemical pathway.

### DETAILED DESCRIPTION OF THE INVENTION

### Aramchol

Aramchol is chemically named 3β-arachidylamido-7α,12α-dihydroxy-5β-cholan-24-oic acid, and is represented by the following chemical structure:

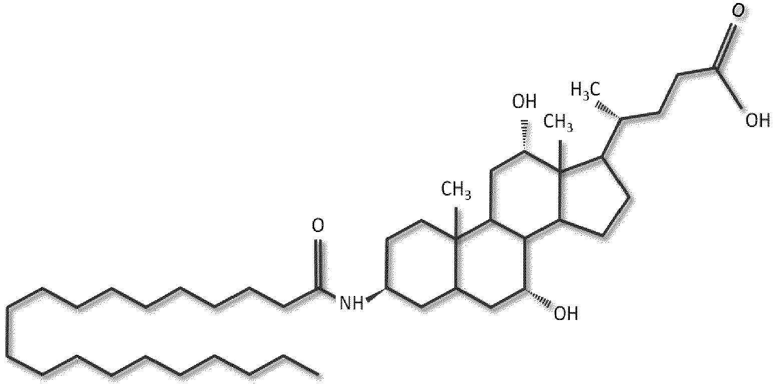

According to an embodiment of the invention, the oral dose for use according to the present invention comprises Aramchol in its free acid form. According to another embodiment of the invention, Aramchol is in its salt form. The salt may be an amine-based salt. The amine-based salt may be selected from meglumine, lysine or tromethamine salts.

### Indications

The invention is based, in part, on the surprising discovery that Aramchol exerts a potent anti-fibrotic effect, independent of its reported activities on fatty liver and steatosis, and reduces fibrosis in various experimental models. Specifically, treatment with Aramchol (5 mg/kg) significantly inhibited the development of toxin-induced cirrhosis, necrosis and liver fibrosis in an in vivo thioacetamide (TAA) model. Aramchol was also found to be unexpectedly superior to obaticholic acid (OCA), which did not induce statistically significant reduction in these parameters under the tested experimental conditions. In addition, Aramchol significantly reduced COL1A1 expression in LX-2 human hepatic stellate cells via PPARy up-regulation. Aramchol was surprisingly found to be effective in reversing established fibrosis, and in reducing the production of collagen specifically in stellate cells.

The present invention thus provides an oral dose of between 350 mg and 1200 mg per day of 3β-arachidylamido-7α, 12α-dihydroxy-5β-cholan-24-oic acid (Aramchol), or a pharmaceutically acceptable salt thereof, as the sole active ingredient for use in treating hepatic fibrosis in a human subject by down regulation of collagen production in the liver of the subject.

Thus, independently from its reported activities on liver metabolism in subjects with NAFLD, Aramchol is surprisingly found herein to be effective in the treatment of new patient populations and patient subpopulations, such as in the treatment of hepatic fibrosis in patients with NAFLD or NASH, treatment of hepatic fibrosis in patients with NAFLD or NASH and advanced fibrosis (i.e. stage 2 or stage 3 fibrosis), treatment of hepatic fibrosis in patient with NAFLD or NASH who have cirrhosis (i.e. stage 4 fibrosis), treatment of hepatic fibrosis caused by contact with drugs, toxins or surgery, and specifically in alleviating hepatic cirrhosis. The invention advantageously provides for the treatment of these new patient populations with enhanced efficacy and/or safety and minimized side effects.

In an embodiment, the human subject is afflicted with Non-Alcoholic Fatty Liver Disease (NAFLD).

In an embodiment, the human subject is afflicted with Non-Alcoholic Steatohepatitis (NASH).

In an embodiment, the human subject is afflicted with NAFLD but not afflicted with Non-Alcoholic Steatohepatitis (NASH). In an embodiment, the human subject has a NAFLD Activity (NAS) Score of at least 4. In an embodiment, the human subject has a NAFLD Activity (NAS) Score of at least 5, at least 6, or at least 7. In an embodiment, the human subject has a ballooning score of at least 1, an inflammation score of at least 1, and a steatosis score of at least 1.

In an embodiment, the human subject is afflicted with Diabetes Mellitus type II or pre-diabetes. One of the following 3 criteria is needed for pre-Diabetes: Fasting Plasma Glucose > 100mg/dl (5.5 mmol/l) or 2hPG following 75g OGTT > 140 (7.8 mmol/l) mg/dl or HbAlc > 5.7%. HbAlc can be repeated at Investigator's discretion.

In an embodiment, the subject's hepatic fibrosis is stage 2, 3, or 4 fibrosis.

In an embodiment, the subject's hepatic fibrosis is stage 1 fibrosis.

In an embodiment, the subject's hepatic fibrosis is stage 1a, stage 1b, or stage 1c fibrosis.

In an embodiment, the human subject has a diet that is high fat and high calorie. As used herein, a high fat, high calorie diet contains at least 4000 calories per day, of which approximately 50% comes from fat.

In an embodiment, the human subject is resistant to lifestyle intervention.

In an embodiment, the human subject is resistant to diet intervention.

In an embodiment, the human subject is naïve to naïve to Aramchol treatment.

In an embodiment, the subject is naïve to NAFLD treatment.

In an embodiment, the oral dose of Aramchol is for use in the treatment of hepatic cirrhosis, portal hypertension, and hepato-fibrotic conditions caused by contact with hepatotoxic chemical substances or by mechanical obstruction.

In another aspect, hepatic fibrosis for treatment in a subject is associated with a disorder other than non-alcoholic liver disease and non-alcoholic steatohepatitis.

In another aspect, the oral dose of Aramchol is useful in treating or inhibiting a disease or condition selected from: alcoholic liver disease, viral hepatitis, parasitic hepatitis, drug-induced hepatitis, toxin-induced hepatitis, primary biliary cirrhosis or congenital hepatic fibrosis.

Known indications suggested for FABAC treatment include those disclosed in US Patents 6,384,024, 6,395,722, 6,589,946, 7,501,403, 8,110,564 and 8,975,246, as detailed herein, and are explicitly excluded in an embodiment. In some embodiments, the subject to be treated is not afflicted with an additional medical condition.

Further, according to an aspect of the present invention, the oral dose of Aramchol is for use in the treatment of hepatic fibrosis in a subject in need thereof, the hepatic fibrosis being caused by contact with a hepatotoxic chemical substance or by mechanical obstruction.

According to embodiments of the present invention, the hepatic fibrosis is caused by a factor selected from chronic alcoholism, malnutrition, hemochromatosis, passive congestion, exposure to poisons or toxins, exposure to drugs, immune reactions, genetically determined sensitivities to a certain substance or infections.

In other embodiments, the hepatic fibrosis is caused by viral hepatitis, syphilis or a parasitic infection. In a particular embodiment, said parasitic infection is Schistosomiasis mansoni or S. japonica infection. In another particular embodiment, the viral hepatitis is associated with is chronic hepatitis C infection.

According to another embodiment, the oral dose of Aramchol is for use in the treatment of hepatic fibrosis associated with a disorder other than non-alcoholic liver disease and non-alcoholic steatohepatitis.

In an embodiment, the subject is not diagnosed with fatty liver. In a particular embodiment, the fibrosis is associated with congenital hepatic fibrosis, a developmental disorder of the liver, marked by formation of irregular broad bands of fibrous tissue containing multiple cysts formed by disordered terminal bile ducts, resulting in vascular constriction and portal hypertension.

Examples for diseases associated with storage or metabolism abnormalities that are characterized by hepatic fibrosis (storage or metabolism hepatic disorders) include α1-Antitrypsin deficiency, copper storage diseases (e.g., Wilson disease), fructosemia, galactosemia, glycogen storage diseases (especially types III, IV, VI, IX, and X), iron-overload syndromes (hemochromatosis), lipid abnormalities (e.g., Gaucher disease), peroxisomal disorders (e.g., Zellweger syndrome), and tyrosinemia.

Examples for infections characterized by liver fibrosis include bacterial infections (e.g., brucellosis), parasitic infections (e.g., echinococcosis), and viral infections (e.g., viral hepatitis including chronic hepatitis B or C). In a particular embodiment, the infection is chronic hepatitis C infection.

According to yet further embodiments, the hepatic fibrosis is associated with contact with a hepatotoxic chemical substance, including alcohol, amiodarone, chlorpromazine, isoniazid, methotrexate, methyldopa, oxyphenisatin, and tolbutamide. In a particular embodiment, said substance is alcohol.

According to further embodiments, the hepatic fibrosis is associated with mechanical obstruction, e.g. scarring due to prior liver surgery.

In another embodiment, the disorder is associated with COL1A1 and/or PPAR-y dysregulation in hepatic stellate calls. In a particular embodiment, said disorder is associated with COL1A1 up-regulation and PPAR-y down-regulation in hepatic stellate calls of said subject.

In another embodiment, said hepatic fibrosis is manifested by portal hypertension and/or hepatic cirrhosis. In a particular embodiment, said fibrosis is manifested by hepatic cirrhosis.

### Administration and dosage form

According to some embodiments, the compound to be administered, i.e., Aramchol, is in the form of a composition comprising a therapeutically effective amount of said compound. As used herein, the term "effective amount" means an amount of Aramchol that is capable of reducing and/or attenuating a disorder or symptom as described herein. The specific dose of Aramchol administered according to this invention will, of course, be determined by the particular circumstances surrounding the case including, for example, the compound administered, the route of administration, the physiological state of the subject, and the severity of the condition being treated.

In an embodiment, the oral dose comprises greater than 350 mg of Aramchol. The oral dose comprises between 350 mg and 1200 mg of Aramchol. In an embodiment, the oral dose comprises 400 mg, 500 mg, 600 mg, 700 mg, 800mg, 900mg, 1000 mg, 1100 mg, or 1200 mg of Aramchol. In an embodiment, the oral dose comprises between 400 mg and 1100 mg, or between 500 mg and 1000 mg, or between 600 mg and 900 mg of Aramchol. In an embodiment, the oral dose comprises 400 mg or 600 mg of Aramchol.

In an embodiment, the oral dose is to be administered daily.

In an embodiment the human subject is any of the human subjects recited hereinabove.

In this invention, the daily dose of Aramchol is between 350 mg and 1200 mg. In an embodiment, the daily dose of Aramchol is 400 mg, 500 mg, 600 mg, 700 mg, 800mg, 900mg, 1000 mg, 1100 mg, or 1200 mg. In an embodiment, the daily dose of Aramchol is between 400 mg and 1100 mg, or between 500 mg and 1000 mg, or between 600 mg and 900 mg. In an embodiment, the daily dose of Aramchol is 400 mg or 600 mg per day.

According to this invention, the composition is formulated for oral administration. Between 350 mg and 1200 mg of Aramchol is administered to the subject per day. In an embodiment, 400 mg, 500 mg, 600 mg, 700 mg, 800mg, 900mg, 1000 mg, 1100 mg, or 1200 mg of Aramchol is administered to the subject per day. In an embodiment, between 400 mg and 1100 mg, or between 500 mg and 1000 mg, or between 600 mg and 900 mg of Aramchol is administered to the subject per day. In an embodiment, 400 mg or 600 mg of Aramchol is administered to the subject per day.

In an embodiment, the oral dose is administered in the morning, in the afternoon, or in the evening.

In an embodiment, the oral dose is administered at the same time as, or within 30 minutes of a meal.

In an embodiment, the meal is breakfast, lunch, or dinner.

In an embodiment, the meal is a high fat meal. A high fat meal is a meal wherein approximately 500 to 600 calories are fat calories.

In an embodiment, the meal is a high calorie meal. A high calorie meal is a meal of approximately 800 to 1000 calories.

In an embodiment, the oral dose of Aramchol is administered with water. In an embodiment, the oral dose of Aramchol is administered with at least 100 or at least 200 mL of water.

In an embodiment, the Aramchol is administered over the course of at least 52 weeks, at least 72 weeks, at least 96 weeks, at least 2 years, at least 3 years, or at least 4 years.

According to some embodiments, oral administration is in the form of hard or soft gelatin capsules, pills, capsules, tablets, including coated tablets, dragees, elixirs, suspensions, liquids, gels, slurries or syrups and controlled release forms thereof Thus, in an embodiment, the oral dose of Aramchol is in the form of a tablet, a capsule, or in a liquid.

Suitable carriers for oral administration are well known in the art. Compositions for oral use can be made using a solid excipient, optionally grinding the resulting mixture, and processing the mixture of granules, after adding suitable auxiliaries as desired, to obtain tablets or dragee cores. Examples of suitable excipients include fillers such as sugars, including lactose, sucrose, mannitol, or sorbitol, cellulose preparations such as, maize starch, wheat starch, rice starch, potato starch, gelatin, gum tragacanth, methyl cellulose, hydroxypropylmethyl-cellulose, and sodium carbomethylcellulose, and/or physiologically acceptable polymers such as polyvinylpyrrolidone (PVP).

If desired, disintegrating agents, such as cross-linked polyvinyl pyrrolidone, agar, or alginic acid or a salt thereof, such as sodium alginate, may be added. Capsules and cartridges of, for example, gelatin for use in a dispenser may be formulated containing a powder mix of the compound and a suitable powder base, such as lactose or starch.

Solid dosage forms for oral administration include capsules, tablets, pills, powders, and granules. In such solid dosage forms, the active compound is admixed with at least one inert pharmaceutically acceptable carrier such as sucrose, lactose, or starch. Such dosage forms can also comprise, as it normal practice, additional substances other than inert diluents, e.g., lubricating, agents. In the case of capsules, tablets and pills, the dosage forms may also comprise buffering agents. Tablets and pills can additionally be prepared with enteric coatings. The term "enteric coating", as used herein, refers to a coating which controls the location of composition absorption within the digestive system. Examples for materials used for enteric coating are fatty acids, waxes, plant fibers or plastics.

Liquid dosage forms for oral administration may further contain adjuvants, such as wetting agents, emulsifying and suspending agents, and sweetening, flavoring and perfuming agents.

According to some embodiments, concomitant treatment with fatty acids such as ethyl eicosapentanoate, eicosapentaenoic acid, and their amides, salts and phospholipids is explicitly excluded. In other embodiment, concomitant treatment with bile acids such as ursodeoxycholic acid and lithocholic acid is excluded. In other embodiments concomitant treatment with vitamin D receptor agonists, acetyl-CoA carboxylase inhibitors, dual PPAR delta/ gamma agonists, and inhibitors of myofibroblast trans-differentiation and activation is excluded.

According to some embodiments, the composition is administered in several dosages over prolonged periods until a sufficient response has been achieved.

As disclosed herein, Aramchol was found to be an unexpectedly potent therapeutic agent, capable of reversing established fibrosis and reducing cirrhosis and collagen synthesis in stellate calls even when used as a single therapeutic agent, in the absence of adjunct therapy. Thus, Aramchol or the pharmaceutically acceptable salt thereof is administered as a sole active ingredient. In another embodiment, the subject is human.

In another embodiment, the Aramchol is in the form of Aramchol free acid. In another embodiment, the Aramchol is in the form of an amine-based salt. In certain particular embodiments, the salt is a meglumine, lysine or tromethamine Aramchol salt.

### Patient outcomes

In an embodiment, treating the subject comprises lack of worsening of the subject's NAFLD Activity (NAS) score.

In an embodiment, treating the subject comprises lack of worsening of the subject's Steatosis, Activity and Fibrosis (SAF) Activity score.

In an embodiment, treating the subject comprises lack of worsening of the subject's fibrosis score.

In an embodiment, the lack of worsening is lack of worsening at 52, 65, 72 or 96 weeks from the commencement of administration of Aramchol.

In an embodiment, the lack of worsening is lack of worsening at 2, 3, or 4 years from the commencement of administration of Aramchol.

In an embodiment, treating the subject treating comprises an improvement of the subject's NAFLD Activity (NAS) score.

In an embodiment, the subject's NAS score is at least 4 at the commencement of administration of Aramchol and the improvement of the subject's NAS score is an improvement of at least 2 points.

In an embodiment, treating the subject comprises an improvement of the subject's Steatosis, Activity and Fibrosis (SAF) Activity Score.

In an embodiment, the subject's SAF Activity score is at least 4 at the commencement of administration of Aramchol and improvement of the subject's SAF Activity score is an improvement of at least 2 points.

In an embodiment, treating the subject comprises an improvement of the subject's fibrosis score.

In an embodiment, the improvement of the subject's fibrosis score is an improvement of 1 grade, or greater than 1 grade. In an embodiment, improvement is improvement at 52, 65, 72, or 96 weeks from the commencement of administration of Aramchol.

In an embodiment, improvement is improvement at 2, 3, or 4 years from the commencement of administration of Aramchol.

In an embodiment, treating the subject comprises inhibiting progression of Non-Alcoholic Fatty Liver Disease (NAFLD).

In an embodiment, inhibiting progression of NAFLD comprises prevention of progression, or reduced progression relative to a patient not treated with Aramchol.

In an embodiment, the human subject is afflicted with Non-Alcoholic Steatohepatitis (NASH) and treating comprises inhibiting progression of NASH.

In an embodiment, inhibiting progression of NASH comprises prevention of progression, or reduced progression relative to a patient not treated with Aramchol.

In an embodiment, treating comprises preventing progression from Non-Alcoholic Fatty Liver Disease (NAFLD) to NASH.

In an embodiment, improvement progression is progression at 2, 4, 8, 24, 40, 52, 65, 72, or 96 weeks from the commencement of administration of Aramchol.

In an embodiment, improvement progression is progression at 2, 3, or 4 years from the commencement of administration of Aramchol.

In an embodiment, the human subject is afflicted with Non-Alcoholic Steatohepatitis (NASH) and the treating comprises NASH resolution in the subject.

In an embodiment, NASH resolution comprises the human subject having a ballooning score of 0 and an inflammation score of 0 or 1.

In an embodiment, treating comprises NASH resolution in the subject at 52, 72, or 96 weeks from the commencement of administration of Aramchol.

In an embodiment, treating comprises NASH resolution in the subject at 2, 3, or 4 years from the commencement of administration of Aramchol.

In an embodiment, treating comprises a reduction in the level of liver triglycerides in the subject relative to the level at the commencement of administration of Aramchol.

In an embodiment, treating comprises a reduction in the ratio of liver triglycerides to water in the subject relative to the ratio at the commencement of administration of Aramchol.

In an embodiment, there is a greater than 10% reduction in ratio of liver triglycerides to water.

In an embodiment, there is a 10% to 40% reduction in ratio of liver triglycerides to water.

In an embodiment, there is a 15% to 35% reduction in ratio of liver triglycerides to water.

In an embodiment, there is a 20% to 30% reduction in ratio of liver triglycerides to water.

In an embodiment, treating comprises:
a. a reduction in the level of Hemoglobin A1C or HOMA-IR;
b. a reduction in the level of Fibrinogen, CK-18, C-reactive protein (CRP), TNFα, IL 6 and fibrosis Tests (NFS);
c. a reduction in the ratio of leptin to adinopectin; or
d. an increase in the level of adinopectin;
   in the subject relative to the level or ratio at the commencement of administration of Aramchol.

In an embodiment, the treating comprises:
a. a reduction in the human subject's body weight relative to the human subject's body weight at the commencement of administration of Aramchol;
b. a reduction in the human subject's waist circumference relative to the human subject's waist circumference at the commencement of administration of Aramchol; or
c. a reduction in the human subject's Fatty Liver Index relative to the human subject's Fatty Liver Index at the commencement of administration of Aramchol.

In an embodiment, the reduction or increase is a reduction or increase at 2, 4, 8, 24, 40, 52, 65, 72, or 96 weeks from the commencement of administration of Aramchol.

In an embodiment, the reduction or increase is a reduction or increase at 2, 3, or 4 years from the commencement of administration of Aramchol.

In another embodiment, administration of an oral dose of Aramchol or a pharmaceutically acceptable salt thereof inhibits collagen synthesis (e.g. COL1A1 expression) in hepatic stellate calls. In another embodiment, administration of an oral dose of Aramchol or a pharmaceutically acceptable salt thereof enhances PPAR-y expression in hepatic stellate calls.

In an embodiment, the oral dose of Aramchol is effective to prevent worsening of NASH in the subject.

In an embodiment, the oral dose of Aramchol is effective to prevent worsening of fibrosis in the subject.

In an embodiment, the oral dose of Aramchol is effective to improve the human subject's NAFLD Activity (NAS) score.

In an embodiment, the oral dose of Aramchol is effective to improve the human subject's NAFLD Activity (NAS) score by at least 2 points.

In an embodiment, the oral dose of Aramchol is effective to improve the human subject's Steatosis, Activity and Fibrosis (SAF) Activity score.

In an embodiment, the oral dose of Aramchol is effective to improve the human subject's Steatosis, Activity and Fibrosis (SAF) Activity score by at least 2 points.

In an embodiment, the oral dose of Aramchol is effective to resolve NASH in the human subject.

In an embodiment, resolving NASH comprises reducing ballooning to a score of 0 and reducing inflammation to a score of 0 or 1.

In an embodiment, the oral dose of Aramchol is effective at 2, 4, 8, 24, 40, 52, 65, 72, or 96 weeks, or 2, 3, or 4 years from the commencement of administration.

In an embodiment, the oral dose of Aramchol is effective to reduce the level of liver triglycerides in the subject relative to the level at the commencement of administration.

In an embodiment, the oral dose of Aramchol is effective to reduce the ratio of liver triglycerides to water in the subject relative to the ratio at the commencement of administration.

In an embodiment, the oral dose of Aramchol is effective to reduce the ratio of liver triglycerides to water in the subject by at least 10% relative to the ratio at the commencement of administration.

In an embodiment, oral dose of Aramchol is effective to reduce the ratio of liver triglycerides to water in the subject by between 10% and 40% relative to the ratio at the commencement of administration.

In an embodiment, oral dose of Aramchol is effective to reduce the ratio of liver triglycerides to water in the subject by between 15% and 35% relative to the ratio at the commencement of administration.

In an embodiment, oral dose of Aramchol is effective to reduce the ratio of liver triglycerides to water in the subject by between 20% and 30% relative to the ratio at the commencement of administration.

In an embodiment, the oral dose of Aramchol is effective to:
a. reduce the level of Hemoglobin A1C or HOMA-IR;
b. reduce the level of Fibrinogen, CK-18, C-reactive protein (CRP), TNFα, IL 6 and fibrosis Tests (NFS);
c. reduce the ratio of leptin to adinopectin; or
d. increase the level of adinopectin;
   in the subject relative to the level or ratio at the commencement of administration.

In an embodiment, the oral dose of Aramchol is effective to:
a. reduce the human subject's body weight relative to the human subject's body weight at the commencement of administration;
b. reduce the human subject's waist circumference relative to the human subject's waist circumference at the commencement of administration; or
c. reduce the human subject's Fatty Liver Index relative to the human subject's Fatty Liver Index at the commencement of administration.

In an embodiment, the oral dose of Aramchol is effective at 2, 4, 8, 24, 40, 52, 65, 72, or 96 weeks, or 2, 3, or 4 years from the commencement of administration.

### Combination Therapy

The present invention also provides an oral dose of between 350 mg and 1200 mg per day of 3β-arachidylamido-7α, 12α-dihydroxy-5β-cholan-24-oic acid (Aramchol), or a pharmaceutically acceptable salt thereof, for use in treating hepatic fibrosis in a human subject by down regulation of collagen production in the liver of the subject, and wherein the subject is further administered a therapeutically effect amount of at least one compound selected from CCR2 chemokine antagonists and CCR5 chemokine antagonists.

The C-C motif chemokine receptor CCR5 is involved in the process by which HIV, the virus that causes AIDS, enters cells. CCR5 receptor antagonists are a class of small molecules that antagonize the CCR5 receptor. Hence, antagonists of this receptor are entry inhibitors and have potential therapeutic applications in the treatment of HIV infections. The C-C chemokine receptor type 2 (CCR2) is a protein that in humans is encoded by the CCR2 gene. This gene encodes two isoforms of a receptor for monocyte chemoattractant protein-1 (CCL2), a chemokine which specifically mediates chemotaxis of cells such as monocytes and macrophages. Cenicriviroc ((S,E)-8-(4-(2-Butoxyethoxy)phenyl)-1-isobutyl-N-(4-(((1-propyl-1H-imidazol-5-yl)methyl)sulfinyl)phenyl)-1,2,3,4-tetrahydrobenzo[b]azocine-5-carboxamide, CAS No. 497223-25-3) is an inhibitor of both CCR2 and CCR5 receptors.

In an embodiment, the CCR2 antagonist is selected from a double-stranded RNA, a compound antagonizing the binding of CCR2 to its ligand, a neutralizing antibody to CCR2, a ligand corresponding to a neutralizing antibody to CCR2, an isolated peptide derived from the sequences of CCR2 or analogs thereof capable of inhibiting CCR2, an antisense nucleic acid, an antagonist microRNA, or an enzymatic RNA molecule.

In an embodiment, the subject is administered a CCR5 antagonist and a CCR2 antagonist, or a dual CCR2/CCR5 antagonist.

In an embodiment, the CCR2/CCR5 dual antagonist is (S,E)-8-(4-(2-Butoxyethoxy)phenyl)-1-isobutyl-N-(4-(((1-propyl-1H-imidazol-5-yl)methyl)sulfinyl)phenyl)-1,2,3,4-tetrahydrobenzo[b]azocine-5-carboxamide (Cenicriviroc) or Cenicriviroc mesylate.

In an embodiment, the subject is further administered a daily dose of 50 to 500 mg of Cenicriviroc, for example a daily dose of 10 to 200 mg of Cenicriviroc, or a daily dose of 50 to 500 mg, 50 to 400 mg, 50 to 300 mg, 50 to 200 mg, or 100 to 200 mg of Cenicriviroc, or a daily dose of 100-200 mg of Cenicriviroc, or a daily dose of 100 or 200 mg of Cenicriviroc.

The embodiments referred to above refer to several drugs being substantially effective in the body at a same time. Several drugs can be administered substantially at the same time, or can be administered at different times but have effect on the body at the same time. For example, this includes administering Aramchol before or subsequently, while functioning of Aramchol in the body is substantially extant.

The administration of two drugs to treat a given condition, such as non-alcoholic fatty liver disease (NAFLD), raises a number of potential problems. In vivo interactions between two drugs are complex. The effects of any single drug are related to its absorption, distribution, and elimination. When two drugs are introduced into the body, each drug can affect the absorption, distribution, and elimination of the other and hence, alter the effects of the other. For instance, one drug may inhibit, activate or induce the production of enzymes involved in a metabolic route of elimination of the other drug. (Guidance for Industry, 1999) Thus, when two drugs are administered to treat the same condition, it is unpredictable whether each will complement, have no effect on, or interfere with the therapeutic activity of the other in a human subject.

Not only may the interaction between two drugs affect the intended therapeutic activity of each drug, but the interaction may increase the levels of toxic metabolites (Guidance for Industry, 1999). The interaction may also heighten or lessen the side effects of each drug. Hence, upon administration of two drugs to treat a disease, it is unpredictable what change will occur in the negative side effect profile of each drug.

Additionally, it is difficult to accurately predict when the effects of the interaction between the two drugs will become manifest. For example, metabolic interactions between drugs may become apparent upon the initial administration of the second drug, after the two have reached a steady-state concentration or upon discontinuation of one of the drugs. (Guidance for Industry, 1999)

### EXAMPLES

Examples are provided below to facilitate a more complete understanding of the invention. The following examples illustrate the exemplary modes of making and practicing the invention.

### Example 1 - Thioacetamide (TAA)-induced fibrosis - model for hepatic cirrhosis

Liver fibrosis was induced in Wistar rats by intraperitoneal injections of TAA (20mg/100 gr body weight) twice per week during 10 weeks. I.p. application of TAA results in hepatic centrolobular necrosis, elevated transaminase activity and robust liver fibrosis. Treatment groups further included co-administration of Aramchol (1 or 5 mg/kg orally) or obaticholic acid (OCA, 5 mg/kg). A control group of saline-treated rats (in the absence of TAA administration) was further included. Rats were then sacrificed, and livers were observed macroscopically for signs of cirrhosis and necrotic lesions, and microscopically, following Masson Goldner staining. The fibrosis score, calculated at a scale of 0-4, was determined for each sample, wherein 0 indicates no fibrosis and 4 indicates advanced fibrosis and cirrhosis.

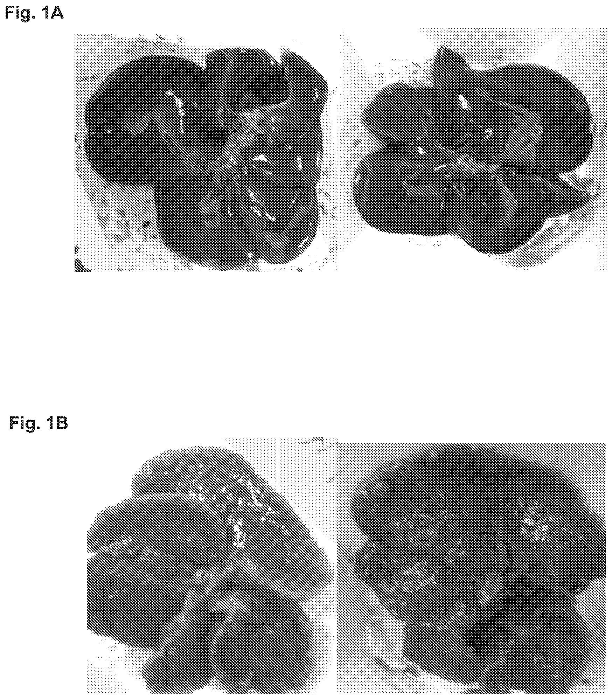

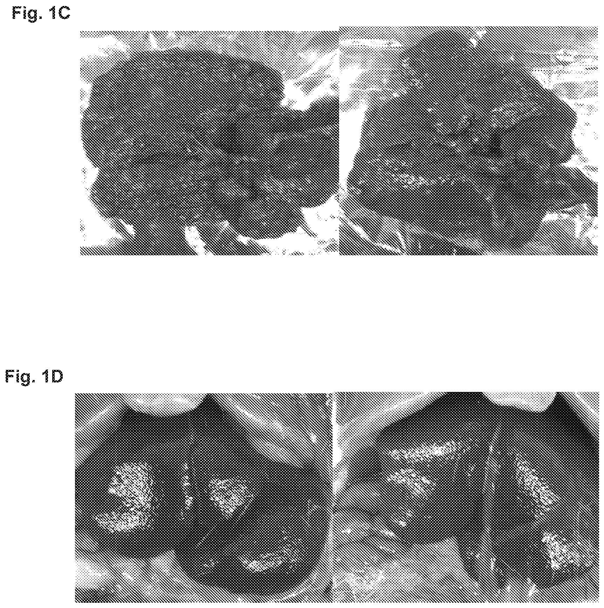

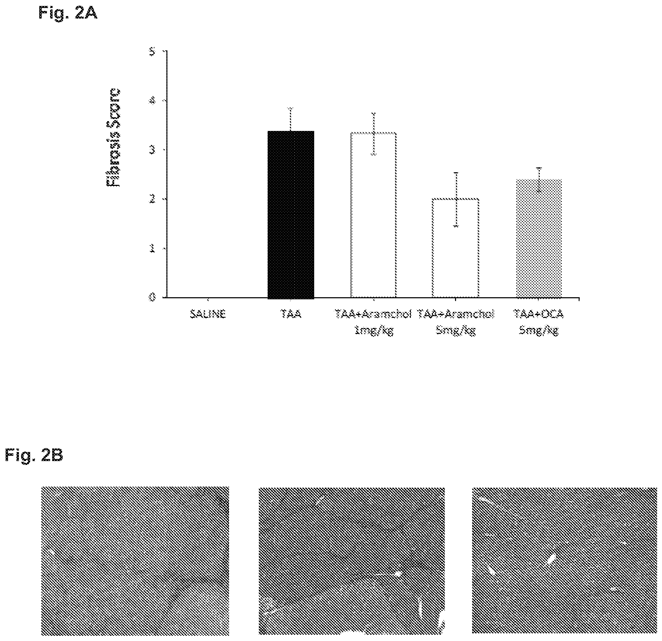

As can be seen in Figures 1-2, treatment with Aramchol (5 mg/kg) significantly prevented TAA induced fibrosis. The treatment reduced significantly the development of necrosis and cirrhosis (Figure 1), as well as the fibrotic score and collagen distribution in the tissue (Figure 2), in a dose-dependent manner. In contradistinction, OCA did not induce statistically significant reduction in these parameters.

Thus, Aramchol was surprisingly found to be a potent anti-fibrotic and anti-cirrhotic agent. Aramchol was also found to be unexpectedly superior to OCA and provide for improved, effective treatment for liver fibrosis.

Cirrhosis and portal hypertension from TAA intoxication may eventually lead to the development of acute liver failure and associated conditions such as hepatic encephalopathy, and the TAA model is also used in evaluating these phenomena. Accordingly, as disclosed herein, Aramchol may also be used in some embodiments for preventing acute or fatal liver failure and/or hepatic or portosystemic encephalopathy, for example toxin-induced liver failure and/or hepatic encephalopathy.

### Example 2 - Inhibition of collagen synthesis in stellate cells

LX2 cells (150.000 cells per well) were plated in DMEM media containing antibiotics, glutamine and bovine fetal serum. After 24 hours incubation, media was changed to 0% serum and incubated for an additional period of 16 hours. Then, Aramchol (10 mM) was added and 24 hours later RNA was extracted with Trizol.

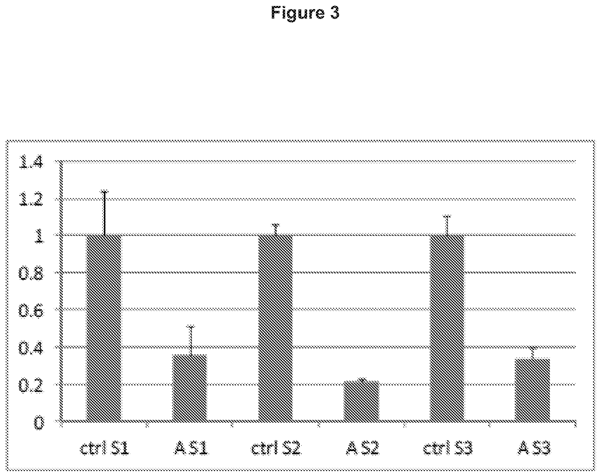

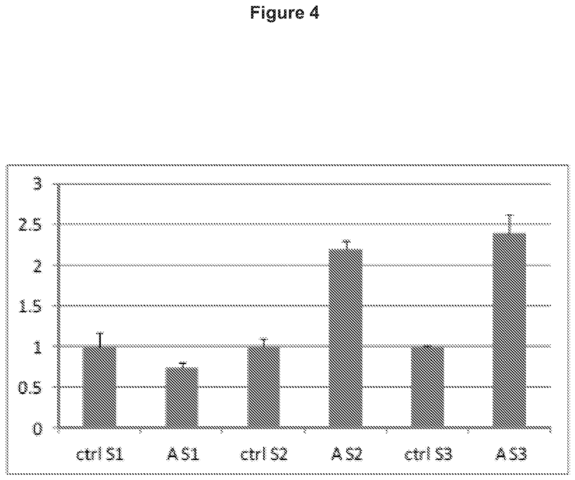

Surprisingly, as can be seen in Figures 3 and 4, COL1A1 expression in LX-2 human hepatic stellate cells was reduced by Aramchol via PPARy up-regulation.

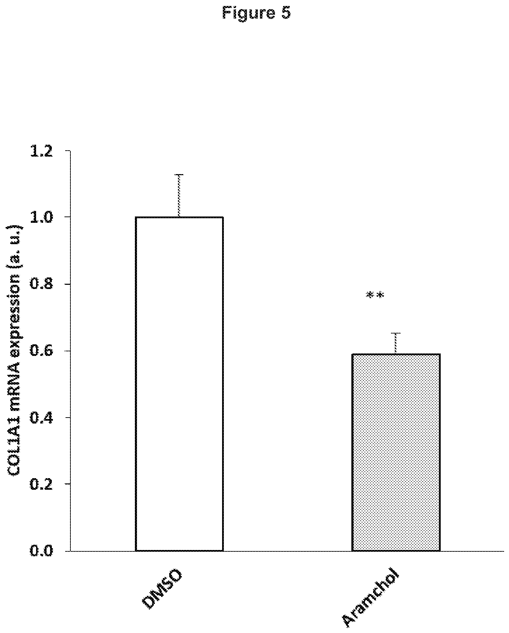

Consistently, Aramchol significantly down regulates collagen production in LX-2 human hepatic stellate cells relative to a DMSO control (Figure 5). Again, Aramchol was surprisingly found to be effective in reducing the production of collagen specifically in stellate cells.

### Example 3 - Aramchol reduces established fibrosis in a MCD diet animal model

The study described below investigates the mechanism of action of Aramchol and its potential effect on fibrosis using the 0.1% methionine- and choline-deficient (0.1MCD) diet mouse model of NASH.

C57BI/6 were fed the Methionine and Choline Deficient (MCD) and control diet and were sacrificed after 4 weeks. The MCD diet induces aminotransferase elevation and changes in hepatic histological features, characterized by steatosis, local inflammation, hepatocyte necrosis and fibrosis. These changes occur rapidly and are morphologically similar to those observed in human NASH. In this study, the MCD diet contained 0.1% methionine to minimize and stabilize weight loss. At the end of the second week, after verification of established NASH, 0.1MCD-fed mice were treated orally by gavage with Aramchol (5 mg/Kg/day) or vehicle (n=10, each condition). Control diet-fed mice were also treated with vehicle for same duration (n=10). At the end of the experiment, blood and liver samples were obtained. A diagram of the experimental design is shown below:

| 0.1MCD diet | 0.1MCD diet + Aramchol^{™} 5mg/kg |
|---|---|
| 14 days | 14 days |
| 28 days | |

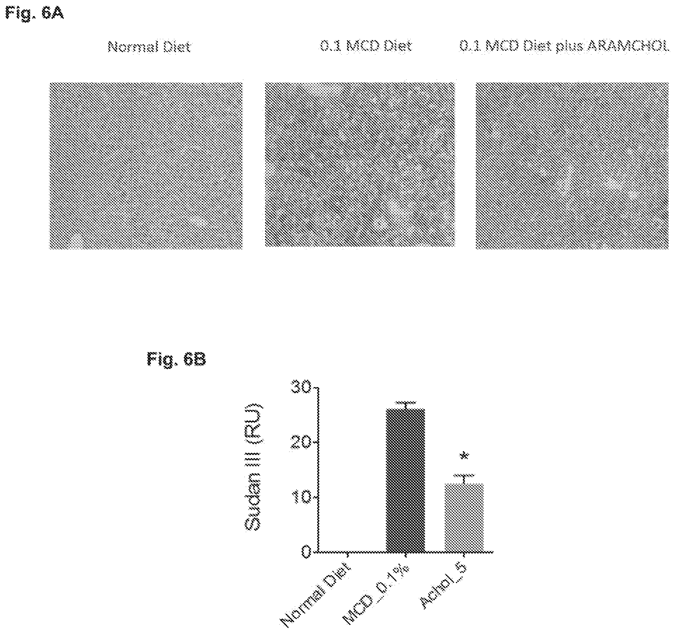

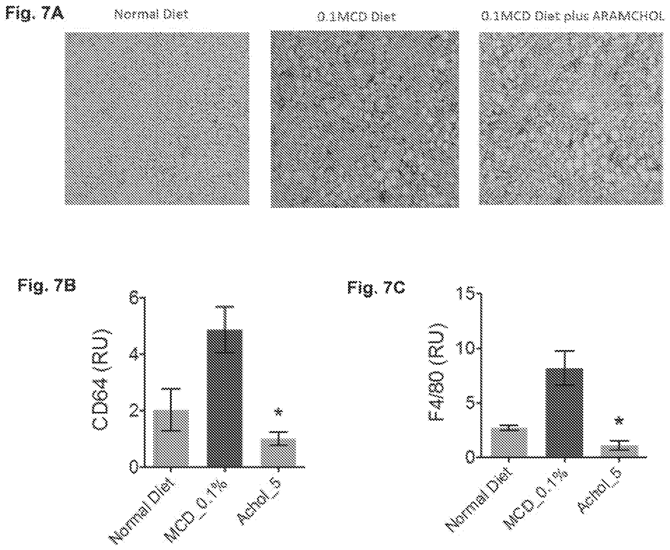

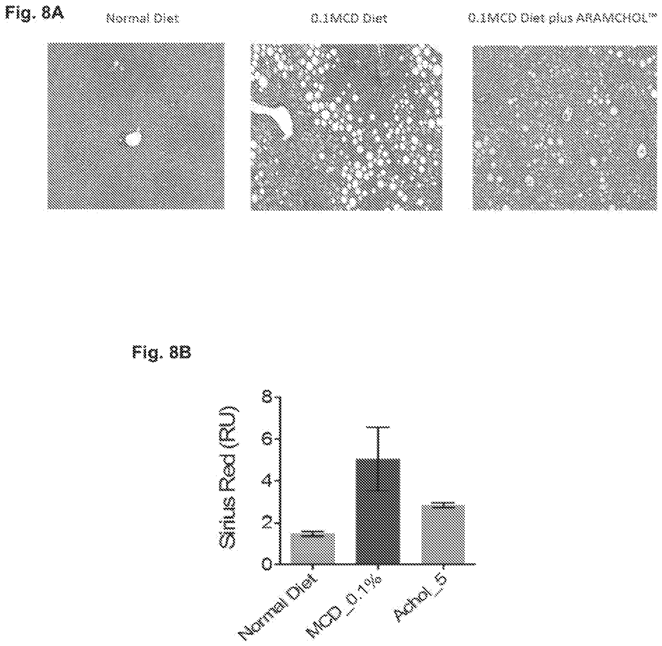

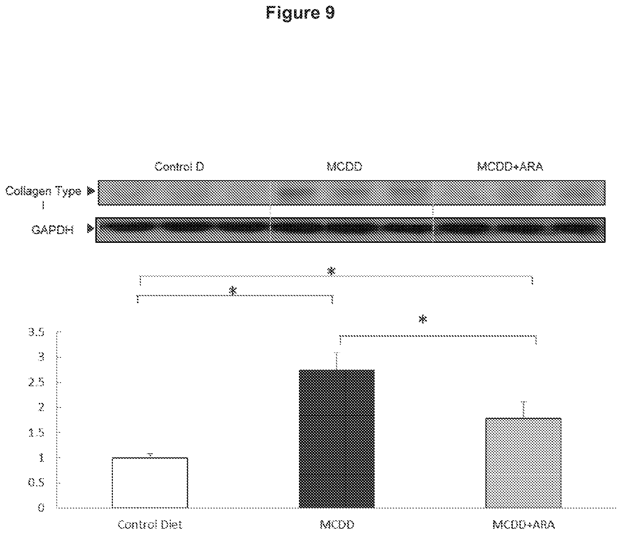

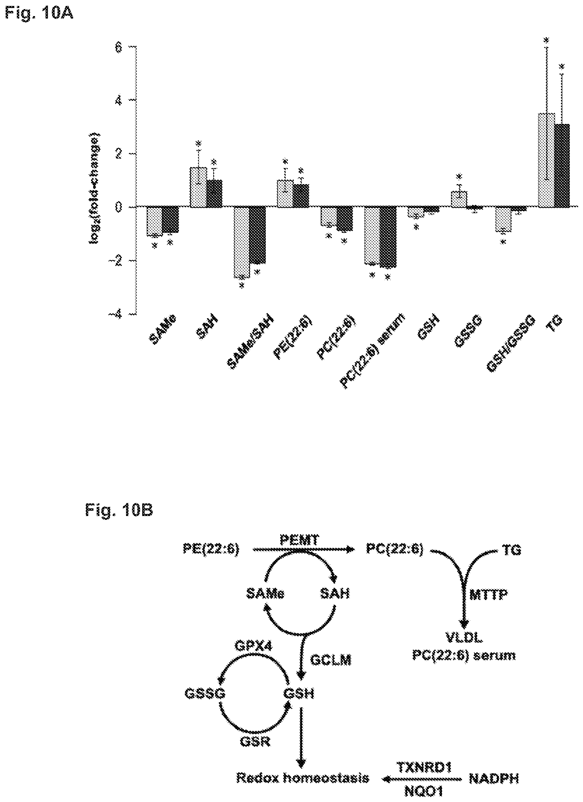

Results from the study showed: 1) treatment with Aramchol significantly down regulates steatosis in the liver (Figure 6); 2) treatment with Aramchol significantly down regulates / normalizes infiltration and activation status of macrophages in the liver (Figure 7); 3) treatment with Aramchol significantly down regulates / normalizes fibrosis in the liver (Figure 8); 4) Aramchol significantly down regulates collagen in the liver (Figure 9); and 5) Aramchol significantly up regulates glutathione and elevates GSH/GSSG ratio in 0.1 % MCD mice (Figure 10).

Additionally, Aramchol treatment further reduced SCD1 activity, which was evidenced by a marked decrease in SCD1 expression, in the FA(16:1)/FA(16:0) ratio and in the total content of monounsaturated FA (MUFA), which led to a reduction in the hepatic content of diglycerides (DG) and TG. Aramchol treatment improved oxidative stress, as shown by the normalization of the GSH/GSSG ratio, a biomarker of the cellular redox potential, and a marked reduction in the content of total oxFA including oxLA, which has been associated with liver injury in human NASH.

The foregoing description of the specific embodiments will so fully reveal the general nature of the invention that others can, by applying current knowledge, readily modify and/or adapt for various applications such specific embodiments without undue experimentation and without departing from the generic concept, and, therefore, such adaptations and modifications should and are intended to be comprehended within the meaning and range of equivalents of the disclosed embodiments. It is to be understood that the phraseology or terminology employed herein is for the purpose of description and not of limitation. The means, materials, and steps for carrying out various disclosed functions may take a variety of alternative forms without departing from the invention.

### Example 4

### Brief Summary

This is a multicenter, Phase IIb, randomized, double blind, placebo-controlled study designed to evaluate the efficacy and safety of two Aramchol doses in subjects that are 18 to 75 years of age, with Non-Alcoholic Steatohepatitis (NASH) confirmed by liver biopsy performed in a period of 6 months before entering the study, with overweight or obesity and who are pre diabetic or type II diabetic.

### Intervention

Drug: Aramchol
Subjects will be administered Aramchol as follows:
   a.One tablet of Aramchol 400 mg and one tablet of matching placebo for Aramchol.
   b.One tablet of Aramchol 400 mg and one tablet of Aramchol 200 mg.
   c.Two tablet of Aramchol matching placebo. The tablets should be taken orally in the morning within 30 min after breakfast with a glass of water (250 ml).
Subjects are allowed to omit study drugs up to 3 consecutive days during the study.
Other Name: Placebo

### Study Arms

Experimental: Aramchol 600mg
   a.One tablet of Aramchol 400 mg and one tablet of Aramchol 200 mg.
   b.lntervention: Drug: Aramchol
Experimental: Aramchol 400mg
   a.One tablet of Aramchol 400 mg and one tablet of matching placebo for Aramchol.
   b.lntervention: Drug: Aramchol
Placebo Comparator: Placebo
   a.Two tablets of Aramchol matching placebo.
   b.Intervention: Drug: Aramchol

### Estimated Enrollment

240

### Inclusion Criteria

Male or female age 18 to 75 years.

BMI between 25kg/m2 to 40kg/m2 or waist circumference between 88 cm to 200 cm for women, and between 102 cm to 200 cm for men. If there is deviation above the upper limit, please consult the MRI center, to ensure that the machine is suitable for the patient.

Known type II Diabetes Mellitus or pre-Diabetes according to American Diabetes Association. One of the following 3 criteria is needed for pre-Diabetes: Fasting Plasma Glucose > 100mg/dl (5.5 mmol/l) or 2hPG following 75g OGTT > 140 (7.8 mmol/l) mg/dl or HbAlc > 5.7%. HbAlc can be repeated at Investigator's discretion.

Histologically proven Steatohepatitis on a diagnostic liver biopsy performed either during screening or within 6 months before screening visit, confirmed by central laboratory reading of the slides.(Steatosis ≥1 + inflammation ≥1 + ballooning ≥1).Total activity NAS score of 4 or more.

Liver fat concentration in the liver of 5.5% or more as measured by NMRS.

Biopsies with an activity NAS score of 4 or more.

Normal synthetic liver function (serum albumin >3.2g/dl, INR 0.8-1.2, conjugated bilirubin < 35 µmol/L).

Understanding the nature of the study and signature of the written informed consent.

Negative pregnancy test at study entry for females of child bearing potential.

Females of child bearing potential practicing reliable contraception throughout the study period (including oral contraceptives) as well as negative pregnancy test at study entry.

Hypertensive patients must be well controlled by stable dose of anti-hypertensive medication for at least 2 months prior to screening.

Patients previously treated with vitamin E (>400IU/day), Polyunsaturated fatty acid (>2g/day) or Ursodeoxycholic acid or fish oil can be included if stopped or at least maintained on stable dose at least 3 months prior to diagnostic liver biopsy (and are not started during the trial). These treatments-dosages are allowed if they were stable for at least 12 months prior to biopsy and can remain stable throughout the study. (Dosages less than the amounts stated above are allowed without washout- or stable-period restrictions).

For patients with type II Diabetes, glycaemia must be controlled (Glycosylated Hemoglobin A1c ≤9%) while any HbAlc change should not exceed 1.5% during 6 months prior to enrolment). Treatments with anti-diabetic medications (except for those mentioned in Exclusion 16) are permitted if glycaemia is self-monitored by the patient. HbAlc can be repeated at Investigator's discretion.

### Exclusion Criteria

### Exclusion Criteria:

Patients with other active (acute or chronic) liver disease other than NASH (e.g. viral hepatitis, unless eradicated at least 3 years prior to screening; genetic hemochromatosis; Wilson disease; alpha 1antitripsin deficiency; alcohol liver disease; drug-induced liver disease) at the time of randomization.
Patients with clinically or histologically documented liver cirrhosis

Known alcohol and/or any other drug abuse or dependence in the last five years.

Known history or presence of clinically significant cardiovascular, gastrointestinal, metabolic other than Diabetes Mellitus, neurologic, pulmonary, endocrine, psychiatric, neoplastic disorder or nephrotic syndrome, that in the opinion of the Investigator warrant exclusion from the study.

Patients with familial (i.e., genetic) hypertriglyceridemia and familial (i.e., genetic) hypercholesterolemia.
History or presence of any disease or condition known to interfere with the absorption distribution, metabolism or excretion of drugs including bile salt metabolites (e.g. inflammatory bowel disease (IBD)), previous intestinal (ileal or colonic) operation, chronic pancreatitis, celiac disease or previous vagotomy. Ongoing Chronic constipation

Patients with heart or brain pacemaker (i.e., implantable neurological devices).

Surgery during the last three month before screening which involved stent implantation of metal devices (e.g. knee, hip etc.)

Weight loss of more than 5% within 6 months prior to randomization.

History of bariatric surgery within 5 years of liver biopsy.

Uncontrolled arterial hypertension.

Women who are pregnant and breast feeding.

Diabetes Mellitus other than type II (type I, endocrinopathy, genetic syndromes etc.).

### Patients with HIV infection.

Daily alcohol intake >20 g/day for women and >30 g/day for men (on average per day) as per medical history.

Treatment with other anti-diabetic medications: GLP-1 receptor agonists and Thiazolidinediones (TZDs), unless started at least 12 months prior to biopsy and on stable dose for 6 months. In case of GLP-1 receptor agonists stopped, it should be at least 6 months before biopsy as per medical history.

SGLT-2 Inhibitors, Metformin, fibrates, statins, insulin, DPP-4 inhibitors and sulfonylurea unless prescribed dose has been stable for the last 6 months prior to the biopsy.

Treatment with Valproic acid, Tamoxifen, Methotrexate, Amiodarone or chronic treatment with anti-cholinergic agents, corticosteroids, high dose estrogen and tetracycline within 12 months prior to the screening visit.

Chronic treatment with antibiotics (e.g. Rifaximin).

Homeopathic and/or alternative treatments. Any treatment should be stopped during the screening period at least 48 hours before randomization.

Uncontrolled hypothyroidism defined as Thyroid Stimulating hormone >2X the upper limit of normal (ULN). Thyroid dysfunction controlled for at least 6 months prior to screening is permitted.

Patients with renal dysfunction eGFR< 40.

Unexplained serum creatine phosphokinase (CPK) >3X the upper limit of normal (UNL). Patients with a reason for CPK elevation may have the measurement repeated prior to randomization; a CPK retest > 3X ULN leads to exclusion.

Patients with condition(s) that makes them unsuitable to perform the NMRS (as determined by the PI or the MRI facility).

Hypersensitivity to Aramchol or to any of the excipients in the tablets.

Hypersensitivity to cholic acid or bile acid sequestrants.

### Detailed Description

This is a multicenter, Phase IIb, randomized, double blind, placebo-controlled study designed to evaluate the efficacy and safety of two Aramchol doses in subjects that are 18 to 75 years of age, with Non-Alcoholic Steatohepatitis (NASH) confirmed by liver biopsy performed in a period of 6 months before entering the study, with overweight or obesity and who are pre diabetic or type II diabetic.

Eligible subjects will be enrolled into three treatments arms: Aramchol 400 and 600 mg tablets and placebo tablets in ratio 2:2:1.

The subjects will be evaluated at study sites for 11 scheduled visits: at screening (visit 1(weeks -4 - 0)), baseline (visit 2 (day 0)), visit 3 (week 2), visit 4 week 4), visit 5 (week 8), visit 6 (week 12), visit 7 (week 24), visit 8 (week 32), visit 9 (week 40) and visit 10 (week 52 - (End of Treatment/early termination visit)). After completion of the study treatment period, the subjects will be followed for an additional period of 13 weeks without study medication (until visit 11 (week 65)).

During the screening period, the severity of the disease will be evaluated with blood tests, liver biopsy and NMRS.

During the study the following assessments will be performed:
a. Vital signs will be measured at each study visit.
b.A physical examination will be performed at the screening visit, 24 weeks, End of Treatment/early termination and week 65 visit.

The following blood tests will be performed: complete blood count (CBC), serum chemistry (including electrolytes, liver enzymes, direct and total bilirubin, glucose, lipid profile which include triglyceride, cholesterol, HDL, LDL and VLDL, CPK, creatinine, urea, albumin, alkaline phosphatase), ESR and urinalysis during the screening visit, baseline, week 2, 4, 8, 24, 40, 52 and 65 (end of follow up) visits. Serology (HBV, HCV and HIV) will be performed during the screening visit. Coagulation (fibrinogen, PT/INR, aPTT) will be measured in screening and baseline, week 24, End of Treatment/early termination and week 65 visits. Insulin (HOMA) will be measured in the screening, week 24 and End of Treatment/early termination visits. HbA1C will be measured in the screening, week 8, 24, 40 and End of Treatment/early termination visits. C reactive protein, Leptin, Adiponectin, CK-18 (M30 and M65), Ferritin, PAI-1, IL-6, TNF-alpha, FGF-19, C4 (7-alpha-hydroxy-4-cholesten-3-one), pool serum Bile Acids, B-hydroxybutyrate and Free Fatty Acids will be measured in baseline visit and end of treatment period. The blood samples taken at these visits, will be tested for possible biomarkers, including, but not limited to, Fetuine A and GDF15. TSH, T3 and T4 will be measured during the screening visit. Beta-hCG in women of childbearing potential will be performed during the screening visit. A serum sample will be collected and kept frozen until study end in case special investigation needs to be performed. This sample will be collected during the screening and visit 10/Early Termination.

Body weight and waist circumference will be measured in screening, baseline, week 24, end of treatment and week 65 visits. Height will be measured during the screening visit.

ECG will be performed during the screening visit, visit 7 (week 24) and end of treatment visits.

All subjects will undergo two NMRS scans, at screening and end of treatment visits.

FibroMax test will be performed only if the investigator thinks it is necessary.

Liver biopsy will be conducted during the screening and end of treatment visit. The biopsy in the screening visit will be performed only if it was not done within the 6 months prior to this visit.

Metabolomics blood test will be performed at the screening, visit 7 and the End-of-Treatment/Early Termination visits. From some consenting patients (about 15) a sample from the liver biopsy will be taken for analysis.

Endothelial Function will be conducted in selected sites. The test will be conducted during the baseline visit before the study treatment will be given and End of Treatment/early termination visit.

Blood sample for Aramchol trough level will be collected (pre-dose) from patients in Israel at baseline (visit 2) week 4 (visit 4), week 12 (visit 6), week 24 (visit 7), week 40 (visit 9), end of treatment (visit 10) and follow up (visit 11). At selected sites in Mexico, USA and Hong Kong one blood sample will be collected (pre-dose) on visit 4 (up to 10 subjects per country) to test for trough Aramchol blood level differences between populations (e.g., African American, Asian, Hispanic).

Blood sample for gene analysis will be taken from all consenting patients during the baseline visit, will be kept frozen and analyzed only at the study end.

Life style questionnaire will be completed in all visits.

Adverse events will be monitored throughout the study.

Concomitant Medications will be monitored throughout the study.

Telephone contacts will be performed on week 16, 20, 28, 36, 44 and 48. An interim safety analysis will be conducted as soon as 120 subjects have completed the follow up period of 24 weeks under study treatment. An independent DSMB will analyze the safety data and recommend a continued course of action. All patients will continue to be treated under the study protocol until conclusion of the analysis will be known.

Safety assessment will include frequency and severity of treatment-emergent AEs, clinically significant laboratory abnormalities, ECG changes and physical examination findings.

### Results

### Primary and Secondary Outcome Measures (400 mg arm)

Treatment with 400 mg of Aramchol significantly reduces liver triglycerides ratio as measured by Magnetic Resonance Spectroscopy (MRS).

Treatment with 400 mg of Aramchol reduces liver triglycerides ratio as measured by Magnetic Resonance Spectroscopy (MRS) by 10-40%.

Treatment with 400 mg of Aramchol reduces liver triglycerides ratio as measured by Magnetic Resonance Spectroscopy (MRS) by 15%-35%.

Treatment with 400 mg of Aramchol reduces liver triglycerides ratio as measured by Magnetic Resonance Spectroscopy (MRS) by 20%-30%.

Treatment with 400 mg of Aramchol results in a significantly higher proportion of subjects having fibrosis improvement (i.e. decrease > or = to 1 point) without a worsening of NASH, compared to subjects treated with a placebo.

Treatment with 400 mg of Aramchol results in a significantly higher proportion of subjects having fibrosis improvement (i.e. decrease > or = to 1 point) without a worsening of NASH, compared to subjects treated with a placebo. The improvement ratio is at least 2 when compared to subjects treated with a placebo.

Treatment with 400 mg of Aramchol results in a significantly higher proportion of subjects with NAS Score improvement (i.e. improvement of at least 2 points) without worsening of fibrosis score, compared to subjects treated with a placebo.

Treatment with 400 mg of Aramchol results in a significantly higher proportion of subjects with SAF Activity score improvement (i.e. improvement of at least 2 points) without worsening of fibrosis score, compared to subjects treated with a placebo.

Treatment with 400 mg of Aramchol results in a significantly higher proportion of subjects with NASH resolution (ballooning of 0, inflammation of 0 or 1) without worsening of fibrosis, compared to subjects treated with a placebo.

### Exploratory Outcome Measures (400 mg arm)

Treatment with 400 mg of Aramchol inhibits worsening of the subject's fibrosis score significantly more than what would be expected based on Aramchol's effect on the subject's liver triglycerides.

Treatment with 400 mg of Aramchol to subjects afflicted with hepatic fibrosis improves the subject's fibrosis score significantly more than what would be expected based on Aramchol's effect on the subject's liver triglycerides.

Treatment with 400 mg of Aramchol to subjects afflicted with stage 1a hepatic fibrosis improves the subject's fibrosis score significantly more than the effect that would be expected based on Aramchol's effect on the subject's liver triglycerides.

Treatment with 400 mg of Aramchol to subjects afflicted with stage 1b hepatic fibrosis improves the subject's fibrosis score significantly more than the effect that would be expected based on Aramchol's effect on the subject's liver triglycerides.

Treatment with 400 mg of Aramchol to subjects afflicted with stage 1c hepatic fibrosis improves the subject's fibrosis score significantly more than the effect that would be expected based on Aramchol's effect on the subject's liver triglycerides.

Treatment with 400 mg of Aramchol to subjects afflicted with stage 2 hepatic fibrosis improves the subject's fibrosis score significantly more than the effect that would be expected based on Aramchol's effect on the subject's liver triglycerides.

Treatment with 400 mg of Aramchol to subjects afflicted with stage 3 hepatic fibrosis improves the subject's fibrosis score significantly more than the effect that would be expected based on Aramchol's effect on the subject's liver triglycerides.

Treatment with 400 mg of Aramchol to subjects afflicted with stage 4 hepatic fibrosis improves the subject's fibrosis score significantly more than the effect that would be expected based on Aramchol's effect on the subject's liver triglycerides.

Treatment with 400 mg of Aramchol to subjects afflicted with hepatic fibrosis improves the subject's SAF score more than what would be expected based on Aramchol's effect on the subject's liver triglycerides.

Treatment with 400 mg of Aramchol to subjects afflicted with hepatic fibrosis results in a significantly higher proportion of subjects without worsening of fibrosis score, compared to subjects afflicted with hepatic fibrosis treated with a placebo.

Treatment with 400 mg of Aramchol to subjects afflicted with hepatic fibrosis results in a significantly higher proportion of subjects having fibrosis improvement (i.e. decrease > or = to 1 point), compared to subjects afflicted with hepatic fibrosis treated with a placebo.

Treatment with 400 mg of Aramchol to subjects afflicted with hepatic fibrosis results in a significantly higher proportion of subjects having fibrosis improvement (i.e. decrease > or = to 1 point) without a worsening of NASH, compared to subjects afflicted with hepatic fibrosis treated with a placebo.

Treatment with 400 mg of Aramchol to subjects afflicted with hepatic fibrosis results in a significantly higher proportion of subjects having fibrosis improvement (i.e. decrease > or = to 1 point) without a worsening of NASH, compared to subjects treated with a placebo. The improvement ratio is at least 2 when compared to subjects afflicted with hepatic fibrosis treated with a placebo.

Treatment with 400 mg of Aramchol results in a significantly higher proportion of subjects with NAS Score improvement (i.e. improvement of at least 2 points) compared to subjects afflicted with hepatic fibrosis treated with a placebo.

Treatment with 400 mg of Aramchol results in a significantly higher proportion of subjects with NAS Score improvement (i.e. improvement of at least 2 points) without worsening of fibrosis score, compared to subjects afflicted with hepatic fibrosis treated with a placebo.

Treatment with 400 mg of Aramchol to subjects afflicted with hepatic fibrosis results in a significantly higher proportion of subjects with SAF Activity score improvement (i.e. improvement of at least 2 points) compared to subjects afflicted with hepatic fibrosis treated with a placebo.

Treatment with 400 mg of Aramchol to subjects afflicted with hepatic fibrosis results in a significantly higher proportion of subjects with SAF Activity score improvement (i.e. improvement of at least 2 points) without worsening of fibrosis score, compared to subjects afflicted with hepatic fibrosis treated with a placebo.

Treatment with 400 mg of Aramchol to subjects afflicted with hepatic fibrosis results in a significantly higher proportion of subjects with NASH resolution (ballooning of 0, inflammation of 0 or 1) compared to subjects afflicted with hepatic fibrosis treated with a placebo.

Treatment with 400 mg of Aramchol to subjects afflicted with hepatic fibrosis results in a significantly higher proportion of subjects with NASH resolution (ballooning of 0, inflammation of 0 or 1) without worsening of fibrosis, compared to subjects afflicted with hepatic fibrosis treated with a placebo.

Treatment with 400 mg of Aramchol to subjects not afflicted with hepatic fibrosis results in a significantly higher proportion of subjects without worsening of fibrosis score, compared to subjects not afflicted with hepatic fibrosis treated with a placebo.

Treatment with 400 mg of Aramchol results in a significantly higher proportion of subjects with NAS Score improvement (i.e. improvement of at least 2 points) compared to subjects not afflicted with hepatic fibrosis treated with a placebo.

Treatment with 400 mg of Aramchol results in a significantly higher proportion of subjects with NAS Score improvement (i.e. improvement of at least 2 points) without worsening of fibrosis score, compared to subjects not afflicted with hepatic fibrosis treated with a placebo.

Treatment with 400 mg of Aramchol to subjects not afflicted with hepatic fibrosis results in a significantly higher proportion of subjects with SAF Activity score improvement (i.e. improvement of at least 2 points) compared to subjects not afflicted with hepatic fibrosis treated with a placebo.

Treatment with 400 mg of Aramchol to subjects not afflicted with hepatic fibrosis results in a significantly higher proportion of subjects with SAF Activity score improvement (i.e. improvement of at least 2 points) without worsening of fibrosis score, compared to subjects not afflicted with hepatic fibrosis treated with a placebo.

Treatment with 400 mg of Aramchol to subjects not afflicted with hepatic fibrosis results in a significantly higher proportion of subjects with NASH resolution (ballooning of 0, inflammation of 0 or 1) compared to subjects not afflicted with hepatic fibrosis treated with a placebo.

Treatment with 400 mg of Aramchol to subjects not afflicted with hepatic fibrosis results in a significantly higher proportion of subjects with NASH resolution (ballooning of 0, inflammation of 0 or 1) without worsening of fibrosis, compared to subjects not afflicted with hepatic fibrosis treated with a placebo.

### Primary and Secondary Outcome Measures (600 mg arm)

Treatment with 600 mg of Aramchol significantly reduces liver triglycerides ratio as measured by Magnetic Resonance Spectroscopy (MRS).

Treatment with 600 mg of Aramchol reduces liver triglycerides ratio as measured by Magnetic Resonance Spectroscopy (MRS) by 10-40%.

Treatment with 600 mg of Aramchol reduces liver triglycerides ratio as measured by Magnetic Resonance Spectroscopy (MRS) by 15%-35%.

Treatment with 600 mg of Aramchol reduces liver triglycerides ratio as measured by Magnetic Resonance Spectroscopy (MRS) by 20%-30%.

Treatment with 600 mg of Aramchol results in a significantly higher proportion of subjects having fibrosis improvement (i.e. decrease > or = to 1 point) without a worsening of NASH, compared to subjects treated with a placebo.

Treatment with 600 mg of Aramchol results in a significantly higher proportion of subjects having fibrosis improvement (i.e. decrease > or = to 1 point) without a worsening of NASH, compared to subjects treated with a placebo. The improvement ratio is at least 2 when compared to subjects treated with a placebo.

Treatment with 600 mg of Aramchol results in a significantly higher proportion of subjects with NAS Score improvement (i.e. improvement of at least 2 points) without worsening of fibrosis score, compared to subjects treated with a placebo.

Treatment with 600 mg of Aramchol results in a significantly higher proportion of subjects with SAF Activity score improvement (i.e. improvement of at least 2 points) without worsening of fibrosis score, compared to subjects treated with a placebo.

Treatment with 600 mg of Aramchol results in a significantly higher proportion of subjects with NASH resolution (ballooning of 0, inflammation of 0 or 1) without worsening of fibrosis, compared to subjects treated with a placebo.

### Exploratory Outcome Measures (600 mg arm)

Treatment with 600 mg of Aramchol inhibits worsening of the subject's fibrosis score significantly more than what would be expected based on Aramchol's effect on the subject's liver triglycerides.

Treatment with 600 mg of Aramchol to subjects afflicted with hepatic fibrosis improves the subject's fibrosis score significantly more than what would be expected based on Aramchol's effect on the subject's liver triglycerides.

Treatment with 600 mg of Aramchol to subjects afflicted with stage 1a hepatic fibrosis improves the subject's fibrosis score significantly more than the effect that would be expected based on Aramchol's effect on the subject's liver triglycerides.

Treatment with 600 mg of Aramchol to subjects afflicted with stage 1b hepatic fibrosis improves the subject's fibrosis score significantly more than the effect that would be expected based on Aramchol's effect on the subject's liver triglycerides.

Treatment with 600 mg of Aramchol to subjects afflicted with stage 1c hepatic fibrosis improves the subject's fibrosis score significantly more than the effect that would be expected based on Aramchol's effect on the subject's liver triglycerides.

Treatment with 600 mg of Aramchol to subjects afflicted with stage 2 hepatic fibrosis improves the subject's fibrosis score significantly more than the effect that would be expected based on Aramchol's effect on the subject's liver triglycerides.

Treatment with 600 mg of Aramchol to subjects afflicted with stage 3 hepatic fibrosis improves the subject's fibrosis score significantly more than the effect that would be expected based on Aramchol's effect on the subject's liver triglycerides.

Treatment with 600 mg of Aramchol to subjects afflicted with stage 4 hepatic fibrosis improves the subject's fibrosis score significantly more than the effect that would be expected based on Aramchol's effect on the subject's liver triglycerides.

Treatment with 600 mg of Aramchol to subjects afflicted with hepatic fibrosis improves the subject's SAF score more than what would be expected based on Aramchol's effect on the subject's liver triglycerides.

Treatment with 600 mg of Aramchol to subjects afflicted with hepatic fibrosis results in a significantly higher proportion of subjects without worsening of fibrosis score, compared to subjects afflicted with hepatic fibrosis treated with a placebo.

Treatment with 600 mg of Aramchol to subjects afflicted with hepatic fibrosis results in a significantly higher proportion of subjects having fibrosis improvement (i.e. decrease > or = to 1 point), compared to subjects afflicted with hepatic fibrosis treated with a placebo.

Treatment with 600 mg of Aramchol to subjects afflicted with hepatic fibrosis results in a significantly higher proportion of subjects having fibrosis improvement (i.e. decrease > or = to 1 point) without a worsening of NASH, compared to subjects afflicted with hepatic fibrosis treated with a placebo.

Treatment with 600 mg of Aramchol to subjects afflicted with hepatic fibrosis results in a significantly higher proportion of subjects having fibrosis improvement (i.e. decrease > or = to 1 point) without a worsening of NASH, compared to subjects treated with a placebo. The improvement ratio is at least 2 when compared to subjects afflicted with hepatic fibrosis treated with a placebo.

Treatment with 600 mg of Aramchol results in a significantly higher proportion of subjects with NAS Score improvement (i.e. improvement of at least 2 points) compared to subjects afflicted with hepatic fibrosis treated with a placebo.

Treatment with 600 mg of Aramchol results in a significantly higher proportion of subjects with NAS Score improvement (i.e. improvement of at least 2 points) without worsening of fibrosis score, compared to subjects afflicted with hepatic fibrosis treated with a placebo.

Treatment with 600 mg of Aramchol to subjects afflicted with hepatic fibrosis results in a significantly higher proportion of subjects with SAF Activity score improvement (i.e. improvement of at least 2 points) compared to subjects afflicted with hepatic fibrosis treated with a placebo.

Treatment with 600 mg of Aramchol to subjects afflicted with hepatic fibrosis results in a significantly higher proportion of subjects with SAF Activity score improvement (i.e. improvement of at least 2 points) without worsening of fibrosis score, compared to subjects afflicted with hepatic fibrosis treated with a placebo.

Treatment with 600 mg of Aramchol to subjects afflicted with hepatic fibrosis results in a significantly higher proportion of subjects with NASH resolution (ballooning of 0, inflammation of 0 or 1) compared to subjects afflicted with hepatic fibrosis treated with a placebo.

Treatment with 600 mg of Aramchol to subjects afflicted with hepatic fibrosis results in a significantly higher proportion of subjects with NASH resolution (ballooning of 0, inflammation of 0 or 1) without worsening of fibrosis, compared to subjects afflicted with hepatic fibrosis treated with a placebo.

Treatment with 600 mg of Aramchol to subjects not afflicted with hepatic fibrosis results in a significantly higher proportion of subjects without worsening of fibrosis score, compared to subjects not afflicted with hepatic fibrosis treated with a placebo.

Treatment with 600 mg of Aramchol results in a significantly higher proportion of subjects with NAS Score improvement (i.e. improvement of at least 2 points) compared to subjects not afflicted with hepatic fibrosis treated with a placebo.

Treatment with 600 mg of Aramchol results in a significantly higher proportion of subjects with NAS Score improvement (i.e. improvement of at least 2 points) without worsening of fibrosis score, compared to subjects not afflicted with hepatic fibrosis treated with a placebo.

Treatment with 600 mg of Aramchol to subjects not afflicted with hepatic fibrosis results in a significantly higher proportion of subjects with SAF Activity score improvement (i.e. improvement of at least 2 points) compared to subjects not afflicted with hepatic fibrosis treated with a placebo.

Treatment with 600 mg of Aramchol to subjects not afflicted with hepatic fibrosis results in a significantly higher proportion of subjects with SAF Activity score improvement (i.e. improvement of at least 2 points) without worsening of fibrosis score, compared to subjects not afflicted with hepatic fibrosis treated with a placebo.

Treatment with 600 mg of Aramchol to subjects not afflicted with hepatic fibrosis results in a significantly higher proportion of subjects with NASH resolution (ballooning of 0, inflammation of 0 or 1) compared to subjects not afflicted with hepatic fibrosis treated with a placebo.

Treatment with 600 mg of Aramchol to subjects not afflicted with hepatic fibrosis results in a significantly higher proportion of subjects with NASH resolution (ballooning of 0, inflammation of 0 or 1) without worsening of fibrosis, compared to subjects not afflicted with hepatic fibrosis treated with a placebo.

### DISCUSSION

Based on studies described herein, Aramchol is surprisingly found to be a potent anti-fibrotic and anti-cirrhotic agent. Aramchol is also found to be unexpectedly superior to OCA and provides improved, effective treatment for liver fibrosis. Accordingly, Aramchol may be used to prevent acute or fatal liver failure and/or hepatic or portosystemic encephalopathy, for example toxin-induced liver failure and/or hepatic encephalopathy.

Furthermore, Aramchol is also surprisingly found to be effective in reversing established fibrosis. Aramchol treatment improves liver histology as determined by a reduction of lipid accumulation (Sudan red staining), fibrosis (Sirius red and SMA staining) and inflammation (F4/80 and CD64 staining). Indeed, Aramchol has an effect on fibrosis in addition to main pathologies of NASH, namely steatosis and inflammation.

Results presented herein show that Aramchol down-regulates collagen production from human stellate cells, the effects of Aramchol are mediated through down regulation of SCD 1 and up regulation of glutathione production, and the effect of Aramchol on fibrosis is mediated via down regulation of steatosis and inflammation as well as directly via down regulation of collagen production from stellate cells. Taken together, information herein supports the effects of Aramchol in human patients as set forth in the claims.

Results analogous to those of Example 4 for 400 mg or 600 mg doses are expected for higher doses of Aramchol that are recited herein.

## Claims

1. An oral dose of between 350 mg and 1200 mg per day of 3β-arachidylamido-7α, 12α-dihydroxy-5β-cholan-24-oic acid (Aramchol), or a pharmaceutically acceptable salt thereof, as the sole active ingredient for use in treating hepatic fibrosis in a human subject by down regulation of collagen production in the liver of the subject.

2. The oral dose for use according to claim 1 wherein the human subject is afflicted with Non-Alcoholic Fatty Liver Disease (NAFLD).

3. The oral dose for use according to claim 2 wherein the human subject is afflicted with Non-Alcoholic Steatohepatitis (NASH), or wherein the human subject is not afflicted with Non-Alcoholic Steatohepatitis (NASH).

4. The oral dose for use according to claim 2 wherein the human subject has a NAFLD Activity (NAS) Score of at least 4, at least 5, at least 6, or at least 7, or wherein the human subject has a ballooning score of at least 1, an inflammation score of at least 1, and a steatosis score of at least 1.

5. The oral dose for use according to any of claims 1-4 wherein the human subject is afflicted with Diabetes Mellitus type II or pre-diabetes.

6. The oral dose for use according to any of claims 1-5 wherein the hepatic fibrosis is stage 1, 2, 3, or 4 fibrosis.

7. The oral dose for use according to any of claims 1-6 wherein the oral dose is 400 mg, 600 mg or 800 mg of Aramchol per day.

8. The oral dose for use according to any of claims 1-7 wherein the Aramchol is administered over the course of at least 52 weeks, at least 72 weeks, at least 96 weeks, at least 2 years, at least 3 years, or at least 4 years.

9. The oral dose for use according to any of claims 1-8 wherein treating comprises inhibiting progression of Non-Alcoholic Fatty Liver Disease (NAFLD) and/or Non-Alcoholic Steatohepatitis (NASH).

10. The oral dose for use according to any of claims 1-9 wherein the human subject is not afflicted with Non-Alcoholic Steatohepatitis (NASH) at commencement of administration and treating comprises preventing progression from Non-Alcoholic Fatty Liver Disease (NAFLD) to NASH; or
wherein the human subject is afflicted with Non-Alcoholic Steatohepatitis (NASH) and the treating comprises NASH resolution in the subject, for example wherein NASH resolution comprises the human subject having a ballooning score of 0 and an inflammation score of 0 or 1.

11. The oral dose for use according to any of claims 1-10 wherein treating comprises a reduction in the ratio of liver triglycerides to water in the subject relative to the ratio at the commencement of administration of Aramchol as measured by MRS, for example a 10% to 40% reduction in ratio of liver triglycerides to water; or
wherein treating comprises:
a) a reduction in the level of Hemoglobin A1C or HOMA-IR;
b) a reduction in the level of Fibrinogen, CK-18, C-reactive protein (CRP), TNFα, IL 6 and fibrosis Tests (NFS);
c) a reduction in the ratio of leptin to adiponectin; or
d) an increase in the level of adiponectin;
in the subject relative to the level or ratio at the commencement of administration of Aramchol, or
e) a reduction in the human subject's body weight relative to the human subject's body weight at the commencement of administration of Aramchol;
f) a reduction in the human subject's waist circumference relative to the human subject's waist circumference at the commencement of administration of Aramchol; or
g) a reduction in the human subject's Fatty Liver Index relative to the human subject's Fatty Liver Index at the commencement of administration of Aramchol.

12. The oral dose for use according to any of claims 1-11 wherein the human subject has a diet that is high fat and high calorie; and/or is resistant to lifestyle intervention or is resistant to diet intervention, wherein a high fat meal is a meal wherein approximately 500 to 600 calories are fat calories and a high calorie meal is a meal of approximately 800 to 1000 calories.

13. An oral dose of between 350 mg and 1200 mg per day of 3β-arachidylamido-7α, 12α-dihydroxy-5β-cholan-24-oic acid (Aramchol), or a pharmaceutically acceptable salt thereof, for use in treating hepatic fibrosis in a human subject by down regulation of collagen production in the liver of the subject, and wherein the subject is further administered a therapeutically effect amount of at least one compound selected from CCR2 chemokine antagonists and CCR5 chemokine antagonists.

14. The oral dose for use according to claim 1, wherein the subject is afflicted with hepatic fibrosis and NASH and has a NAS Score of at least 5, at least 6, or at least 7, a ballooning score of at least 1, an inflammation score of at least 1, and a steatosis score of at least 1, and wherein the subject is administered a dose of 400 mg or 600 mg or 800 mg per day of the Aramchol or salt thereof.

## Patentansprüche

1. Orale Dosis von 350 mg bis 1200 mg pro Tag 3β-Arachidylamido-7a, 12α-Dihydroxy-5β-cholan-24-oinsäure (Aramchol) oder eines pharmazeutisch annehmbaren Salzes davon als dem einzigen aktiven Inhaltsstoff zur Anwendung beim Behandeln einer hepatischen Fibrose bei einem humanen Subjekt durch Herabregulierung der Kollagenproduktion in der Leber.

2. Orale Dosis nach Anspruch 1, wobei das humane Subjekt an einer nichtalkoholischen Fettlebererkrankung (*Non-Alcoholic Fatty Liver Disease, NAFLD*) leidet.

3. Orale Dosis nach Anspruch 2, wobei das humane Subjekt an einer nichtalkoholischen Steatohepatitis (*Non-Alcoholic SteatoHepatitis, NASH*) leidet oder wobei das humane Subjekt nicht an einer nichtalkoholischen Steatohepatitis (NASH) leidet.

4. Orale Dosis zur Anwendung nach Anspruch 2, wobei das humane Subjekt einen NAFLD-Aktivitäts-Score (NAS) von mindestens 4, mindestens 5, mindestens 6 oder mindestens 7 hat oder wobei das humane Subjekt einen Ballonnierungs-Score von mindestens 1, einen Entzündungs-Score von mindestens 1 und einen Steatose-Score von mindestens 1 hat.

5. Orale Dosis zur Anwendung nach einem der Ansprüche 1-4, wobei das humane Subjekt an Diabetes mellitus (Diabetes Typ II) oder Prädiabetes leidet.

6. Orale Dosis zur Anwendung nach einem der Ansprüche 1-5, wobei die hepatische Fibrose eine Fibrose im Stadium 1, 2, 3 oder 4 ist.

7. Orale Dosis zur Anwendung nach einem der Ansprüche 1-6, wobei die orale Dosis 400 mg, 600 mg oder 800 mg Aramchol pro Tag ist.

8. Orale Dosis zur Anwendung nach einem der Ansprüche 1-7, wobei das Aramchol über eine Dauer von mindestens 52 Wochen, von mindestens 72 Wochen, von mindestens 96 Wochen, von mindestens 2 Jahren, von mindestens 3 Jahren oder von mindestens 4 Jahren verabreicht wird.

9. Orale Dosis zur Anwendung nach einem der Ansprüche 1-8, wobei Behandeln ein Hemmen der Progredienz einer nichtalkoholischen Fettlebererkrankung (NAFLD) und/oder nichtalkoholischen Steatohepatitis (NASH) umfasst.

10. Orale Dosis zur Anwendung nach einem der Ansprüche 1-9, wobei das humane Subjekt zu Beginn der Verabreichung nicht an einer nichtalkoholischen Fettlebererkrankung (NASH) leidet und wobei Behandeln ein Vorbeugen gegen eine Progredienz einer nichtalkoholischen Fettlebererkrankung (NAFLD) in eine NASH umfasst, oder
wobei das humane Subjekt an einer nichtalkoholischen Steatohepatitis (NASH) leidet und Behandeln eine Behebung von NASH bei dem Subjekt umfasst, wobei eine NASH-Behebung beispielsweise umfasst, dass das humane Subjekt einen Ballonnierungs-Score von 0 und einen Entzündungs-Score von 0 oder 1 hat.

11. Orale Dosis zur Anwendung nach einem der Ansprüche 1-10, wobei Behandeln eine Reduzierung des Verhältnisses von Leber-Triglyzeriden zu Wasser bei dem Subjekt in Relation zu dem Verhältnis zu Beginn der Aramchol-Verabreichung gemäß einer MRS-Messung umfasst, so beispielsweise eine Reduzierung des Verhältnisses von Leber-Triglyzeriden zu Wasser um 10 % bis 40 %; oder wobei Behandeln wie folgt umfasst:
a) eine Reduzierung des Spiegels von Hämoglobin A1C oder HOMA-IR;
b) eine Reduzierung des Spiegels von Fibrinogen, CK-18, C-reaktivem Protein (CRP), TNFα, IL 6 und Fibrose-Tests (NFS);
c) eine Reduzierung des Verhältnisses von Leptin zu Adiponektin; oder
d) einen Anstieg des Spiegels von Adiponektin;
bei dem Subjekt in Relation zu dem Spiegel oder Verhältnis zu Beginn der Aramchol-Verabreichung; oder
e) eine Reduzierung des Körpergewichts des humanen Subjekts in Relation zu dem Körpergewicht des humanen Subjekts zu Beginn der Aramchol-Verabreichung;
f) eine Reduzierung des Taillenumfangs des humanen Subjekts in Relation zu dem Taillenumfang des humanen Subjekts zu Beginn der Aramchol-Verabreichung; oder
g) eine Reduzierung des Fettleber-Indexes in Relation zu dem Fettleber-Index des humanen Subjekts zu Beginn der Aramchol-Verabreichung.

12. Orale Dosis zur Anwendung nach einem der Ansprüche 1-11, wobei das humane Subjekt eine hochfetthaltige und hochkalorienhaltige Ernährungsweise hat und/oder gegen Eingriffe in die normale Lebensführung oder gegen Eingriffe in die normale Ernährungsweise resistent ist, wobei eine hochfetthaltige Mahlzeit eine Mahlzeit ist, bei der etwa 500 bis 600 Kalorien Fettkalorien sind und wobei eine hochkalorienhaltige Mahlzeit eine Mahlzeit von etwa 800 bis 1000 Kalorien ist.

13. Orale Dosis von 350 mg bis 1200 mg pro Tag 3β-Arachidylamido-7α, 12α-Dihydroxy-5β-cholan-24-oinsäure (Aramchol) oder eines pharmazeutisch annehmbaren Salzes davon zur Anwendung beim Behandeln einer hepatischen Fibrose bei einem humanen Subjekt durch Herabregulierung der Kollagenproduktion in der Leber des Subjekts und wobei dem Subjekt ferner eine therapeutisch wirksame Menge mindestens einer Verbindung, die unter CCR2-Chemokin-Antagonisten und CCRS-Chemokin-Antagonisten ausgewählt ist, verabreicht wird.

14. Orale Dosis zur Anwendung nach Anspruch 1, wobei das Subjekt an einer hepatischen Fibrose NASH leidet und einen NAS-Score von mindestens 5, mindestens 6 oder mindestens 7, einen Ballonnierungs-Score von mindestens 1, einen Entzündungs-Score von mindestens 1 und einen Steatose-Score von mindestens 1 hat und wobei dem Subjekt eine Dosis in Höhe von 400 mg oder 600 mg oder 800 mg pro Tag Aramchol oder von einem Salz davon verabreicht wird.

## Revendications

1. Dose orale comprise entre 350 mg et 1200 mg par jour d'acide 3β-arachidylamido-7α, 12α-dihydroxy-5 β-cholan-24-oïque (Aramchol), ou d'un sel pharmaceutiquement acceptable de celui-ci, comme seul ingrédient actif pour une utilisation dans le traitement de la fibrose hépatique chez un sujet humain par régulation négative de la production de collagène dans le foie du sujet.

2. Dose orale pour une utilisation selon la revendication 1, dans laquelle le sujet humain est atteint d'une stéatose hépatique non alcoolique (NAFLD).

3. Dose orale pour une utilisation selon la revendication 2, dans laquelle le sujet humain est atteint de stéatohépatite non alcoolique (NASH), ou dans laquelle le sujet humain n'est pas atteint de stéatohépatite non alcoolique (NASH).

4. Dose orale pour une utilisation selon la revendication 2, dans laquelle le sujet humain a un score d'activité NAFLD (NAS) d'au moins 4, d'au moins 5, d'au moins 6 ou d'au moins 7, ou dans laquelle le sujet humain a un score de ballonnement d'au moins 1, un score d'inflammation d'au moins 1 et un score de stéatose d'au moins 1.

5. Dose orale pour une utilisation selon l'une quelconque des revendications 1 à 4, dans laquelle le sujet humain est atteint de diabète sucré de type II ou de prédiabète.

6. Dose orale pour une utilisation selon l'une quelconque des revendications 1 à 5, dans laquelle la fibrose hépatique est une fibrose de stade 1, 2, 3 ou 4.

7. Dose orale pour une utilisation selon l'une quelconque des revendications 1 à 6, dans laquelle la dose orale est de 400 mg, 600 mg ou 800 mg d'Aramchol par jour.

8. Dose orale pour une utilisation selon l'une quelconque des revendications 1 à 7, dans laquelle l'Aramchol est administré sur une période d'au moins 52 semaines, d'au moins 72 semaines, d'au moins 96 semaines, d'au moins 2 ans, d'au moins 3 ans, ou d'au moins 4 ans.

9. Dose orale pour une utilisation selon l'une quelconque des revendications 1 à 8, dans laquelle le traitement comprend l'inhibition de la progression de la stéatose hépatique non alcoolique (NAFLD) et/ou de la stéatohépatite non alcoolique (NASH).

10. Dose orale pour une utilisation selon l'une quelconque des revendications 1 à 9, dans laquelle le sujet humain n'est pas atteint de stéatohépatite non alcoolique (NASH) au début de l'administration et le traitement comprend la prévention de la progression de la stéatose hépatique non alcoolique (NAFLD) vers la NASH ; ou
le sujet humain est atteint de stéatohépatite non alcoolique (NASH) et le traitement comprend une résolution de la NASH chez le sujet, par exemple dans lequel la résolution de la NASH concerne le sujet humain ayant un score de ballonnement de 0 et un score d'inflammation de 0 ou 1.

11. Dose orale pour une utilisation selon l'une quelconque des revendications 1 à 10, dans laquelle le traitement comprend une réduction du rapport des triglycérides hépatiques à l'eau chez le sujet par rapport au rapport au début de l'administration d'Aramchol tel que mesuré par MRS, par exemple un réduction de 10 à 40 % du rapport triglycérides hépatiques/eau ; ou
dans lequel le traitement comprend :
a) une réduction du taux d'hémoglobine A1C ou HOMA-IR ;
b) une réduction du taux de fibrinogène, de CK-18, de protéine C-réactive (CRP), de TNFα, d'IL 6 et de tests de fibrose (NFS) ;
c) une réduction du rapport leptine/adiponectine ; ou
d) une augmentation du taux d'adiponectine ;
chez le sujet par rapport au niveau ou au rapport au début de l'administration d'Aramchol ; ou
e) une réduction du poids corporel du sujet humain par rapport au poids corporel du sujet humain au début de l'administration d'Aramchol ;
f) une réduction du tour de taille du sujet humain par rapport au tour de taille du sujet humain au début de l'administration d'Aramchol ; ou
g) une réduction de l'indice de stéatose hépatique du sujet humain par rapport à l'indice de stéatose hépatique du sujet humain au début de l'administration d'Aramchol.

12. Dose orale pour une utilisation selon l'une quelconque des revendications 1 à 11, dans laquelle le sujet humain suit un régime alimentaire riche en graisses et en calories ; et/ou résiste à une intervention sur son mode de vie ou résiste à une intervention sur son régime alimentaire; un repas riche en graisses étant un repas dans lequel environ 500 à 600 calories sont des calories grasses et un repas riche en calories est un repas d'environ 800 à 1000 calories.

13. Dose orale comprise entre 350 mg et 1200 mg par jour d'acide 3β-arachidylamido-7α, 12α-dihydroxy-5 α-cholan-24-oïque (Aramchol) ou d'un sel pharmaceutiquement acceptable de celui-ci, pour une utilisation dans le traitement de la fibrose hépatique chez un sujet humain par régulation négative de la production de collagène dans le foie du sujet, et dans laquelle le sujet reçoit en outre une quantité à effet thérapeutique d'au moins un composé choisi parmi les antagonistes de chimiokine CCR2 et les antagonistes de chimiokine CCR5.

14. Dose orale pour une utilisation selon la revendication 1, dans laquelle le sujet est atteint de fibrose hépatique et de NASH et a un score NAS d'au moins 5, d'au moins 6 ou d'au moins 7, un score de ballonnement d'au moins 1, un score d'inflammation d'au moins 1 et un score de stéatose d'au moins 1, et dans lequel le sujet reçoit une dose de 400 mg ou 600 mg ou 800 mg par jour d'Aramchol ou de son sel.
